# NEUE EUROPÄISCHE PATENTSCHRIFT

(11) **EP 0 972 198 B2**
(45) Veröffentlichungstag und Bekanntmachung des Hinweises auf die Entscheidung über den Einspruch: **11.12.2013**
(45) Hinweis auf die Patenterteilung: 20.11.2002
(21) Anmeldenummer: 98912441.7
(22) Anmeldetag: 05.03.1998
(51) Int. Cl.: G01N 33/569, C07K 16/10

(54) **VERFAHREN ZUR SIMULTANEN BESTIMMUNG VON HIV-ANTIGENEN UND HIV-ANTIKÖRPERN**
METHOD FOR SIMULTANEOUS DETECTION OF HIV ANTIGENS AND HIV ANTIBODIES
PROCEDE DE DETECTION SIMULTANEE D'ANTIGENES DE VIH ET D'ANTICORPS DE VIH

(30) Priorität: 10.03.1997 DE 19709762; 01.07.1997 DE 19727943
(43) Veröffentlichungstag der Anmeldung: 19.01.2000
(73) Patentinhaber: Roche Diagnostics GmbH, 68305 Mannheim (DE)
(72) Erfinder: DONIE, Frederic, D-82377 Penzberg (DE); FAATZ, Elke, D-82386 Huglfing (DE); UPMEIER, Barbara, D-82393 Iffeldorf (DE); HOESS, Eva, D-81476 München (DE); Saman, Eric, 2880 Bornem (BE); Buyse, Marie-Ange, 9820 Melsen (BE)
(74) Vertreter: Weiss, Wolfgang
(86) Internationale Anmeldenummer: PCT/EP1998/001235
(87) Internationale Veröffentlichungsnummer: WO 1998/040744

(56) Entgegenhaltungen:
- EP-A- 0 265 851
- EP-A- 0 386 713
- EP-A- 0 595 211
- EP-A1- 0 345 375
- WO-A-93/21346
- FILICE ET AL: "Sensitivity and specificity of anti HIV Elisa employing recombinant (p24, p66, gp120) and synthetic (gp41) viral antigenic peptides" MICROBIOLOGICA, Bd. 14, Nr. 3, Juli 1991, Seiten 185-194, XP002072105
- HASHIDA ET AL J.OF VIROLOGICAL METHODS Bd. 62, 1996, Seiten 43 - 53
- 'AIDS 7(1)' CONSTANTINE 1993, Seiten 1 - 13
- ZAAJER ET AL THE LANCET 340 1992, Seiten 770 - 772
- SIMON ET AL THE LANCET 340 1992, Seiten 1541 - 1542
- COURCOUCE ET AL. AIDS 6(1) 1992, Seiten 1548 - 1550

### SEQUENZPROTOKOLL

### (1) ALLGEMEINE ANGABEN:

(i) ANMELDER:
   (A) NAME: Boehringer Mannheim GmbH
   (B) STRASSE: Sandhoferstr, 116
   (C) ORT: Mannheim
   (E) LAND: DE
   (F) POSTLEITZAHL: 68305
   (G) TELEFON: 06217593215
   (H) TELEFAX: 06217594457
(ii) BEZEICHNUNG DER ERFINDUNG: Kombitest HIV
(iii) ANZAHL DER SEQUENZEN: 36
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30 (EPA)

### (2) ANGABEN ZU SEQ ID NO: 1:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 1:
   Asp Lys Gly Asn Ser Ser Gln Val Ser Gln Asn Tyr Pro Ile Val
    1               5                  10                  15
   Gln Asn Leu Gln Gly Gln Met Val His Gln
                   20                  25

### (2) ANGABEN ZU SEQ ID NO: 2:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 2:
   Asn Tyr Pro Ile Val Gln Asn Leu Gln Gly Gln Met Val His Gln
    1              5                   10                  15
   Ala Ile Ser Pro Arg Thr Leu Asn Ala Trp
                   20                  25

### (2) ANGABEN ZU SEQ ID NO: 3:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 3:
   Gln Met Val His Gln Ala Ile Ser Pro Arg Thr Leu Asn Ala Trp
    1              5                   10                  15
   Val Lys Val Ile Glu Glu Lys Ala Phe Ser
                   20                  25

### (2) ANGABEN ZU SEQ ID NO: 4:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 4:
   Thr Leu Asn Ala Trp Val Lys Val Ile Glu Glu Lys Ala Phe Ser
   1 5 10 15
   Pro Glu Val Ile Pro Met Phe Ser Ala Leu
   20 25

### (2) ANGABEN ZU SEQ ID NO: 5:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 5:
   Glu Lys Ala Phe Ser Pro Glu Val Ile Pro Met Phe Ser Ala Leu
   1 5 10 15
   Ser Glu Gly Ala Thr Pro Gln Asp Leu Asn
   20 25

### (2) ANGABEN ZU SEQ ID NO: 6:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 6:
   Met Phe Ser Ala Leu Ser Glu Gly Ala Thr Pro Gln Asp Leu Asn
   1 5 10 15
   Thr Met Leu Asn Thr Val Gly Gly His Gln
   20 25

### (2) ANGABEN ZU SEQ ID NO: 7:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 7:
   Pro Gln Asp Leu Asn Thr Met Leu Asn Thr Val Gly Gly His Gln
    1               5                  10                  15
   Ala Ala Met Gln Met Leu Lys Glu Thr Ile
                   20                  25

### (2) ANGABEN ZU SEQ ID NO: 8:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 8:
   Val Gly Gly His Gln Ala Ala Met Gln Met Leu Lys Glu Thr Ile
    1               5                  10                  15
   Asn Glu Glu Ala Ala Glu Trp Asp Arg Val
                   20                  25

### (2) ANGABEN ZU SEQ ID NO: 9:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 9:
   Leu Lys Glu Thr Ile Asn Glu Glu Ala Ala Glu Trp Asp Arg Val
    1               5                  10                  15
   His Pro Val His Ala Gly Pro Ile Ala Pro
                   20                  25

### (2) ANGABEN ZU SEQ ID NO: 10:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 10:
   Glu Trp Asp Arg Val His Pro Val His Ala Gly Pro Ile Ala Pro
    1               5                  10                  15
   Gly Gln Met Arg Glu Pro Arg Gly Ser Asp
                   20                  25

### (2) ANGABEN ZU SEQ ID NO: 11:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 11:
   Gly Pro Ile Ala Pro Gly Gln Met Arg Glu Pro Arg Gly Ser Asp
   1               5                   10                  15
   Ile Ala Gly Thr Thr Ser Thr Leu Gln Glu
               20                  25

### (2) ANGABEN ZU SEQ ID NO: 12:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 12:
   Pro Arg Gly Ser Asp Ile Ala Gly Thr Thr Ser Thr Leu Gln Glu
   1               5                   10                  15
   Gln Ile Gly Trp Met Thr Asn Asn Pro Pro
               20                  25

### (2) ANGABEN ZU SEQ ID NO: 13:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 13:
   Ser Thr Leu Gln Glu Gln Ile Gly Trp Met Thr Asn Asn Pro Pro
   1               5                   10                  15
   Ile Pro Val Gly Glu Ile Tyr Lys Arg Trp
               20                  25

### (2) ANGABEN ZU SEQ ID NO: 14:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 14:
   Thr Asn Asn Pro Pro Ile Pro Val Gly Glu Ile Tyr Lys Arg Trp
    1               5                   10                  15
   Ile Ile Leu Gly Leu Asn Lys Ile Val Arg
                   20                  25

### (2) ANGABEN ZU SEQ ID NO: 15:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 15:
   Ile Tyr Lys Arg Trp Ile Ile Leu Gly Leu Asn Lys Ile Val Arg
    1               5                   10                  15
   Met Tyr Ser Pro Val Ser Ile Leu Asp Ile
                   20                  25

### (2) ANGABEN ZU SEQ ID NO: 16:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 16:
   Asn Lys Ile Val Arg Met Tyr Ser Pro Val Ser Ile Leu Asp Ile
   1               5                   10                  15
   Arg Gln Gly Pro Lys Glu Pro Phe Arg Asp
                   20                  25

### (2) ANGABEN ZU SEQ ID NO: 17:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 17:
   Ser Ile Leu Asp Ile Arg Gln Gly Pro Lys Glu Pro Phe Arg Asp
   1               5                   10                  15
   Tyr Val Asp Arg Phe Tyr Lys Thr Leu Arg
                   20                  25

### (2) ANGABEN ZU SEQ ID NO: 18:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 18:
   Glu Pro Phe Arg Asp Tyr Val Asp Arg Phe Tyr Lys Thr Leu Arg
   1               5                   10                  15
   Ala Glu Gln Ala Ser Gln Glu Val Lys Asn
                   20                  25

### (2) ANGABEN ZU SEQ ID NO: 19:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 19:
   Tyr Lys Thr Leu Arg Ala Glu Gln Ala Ser Gln Glu Val Lys Asn
    1               5                  10                  15
   Trp Met Thr Glu Thr Leu Leu Val Gln Asn
                   20                  25

### (2) ANGABEN ZU SEQ ID NO: 20:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 20:
   Gln Glu Val Lys Asn Trp Met Thr Glu Thr Leu Leu Val Gln Asn
    1               5                  10                  15
   Ala Asn Pro Asp Cys Lys Thr Ile Leu Lys
                   20                  25

### (2) ANGABEN ZU SEQ ID NO: 21:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 21:
   Leu Leu Val Gln Asn Ala Asn Pro Asp Cys Lys Thr Ile Leu Lys
   1               5                   10                  15
   Ala Leu Gly Pro Ala Ala Thr Leu Glu Glu
                   20                  25

### (2) ANGABEN ZU SEQ ID NO: 22:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 22:
   Lys Thr Ile Leu Lys Ala Leu Gly Pro Ala Ala Thr Leu Glu Glu
   1               5                   10                  15
   Met Met Thr Ala Cys Gln Gly Val Gly Gly
                   20                  25

### (2) ANGABEN ZU SEQ ID NO: 23:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 23:
   Ala Thr Leu Glu Glu Met Met Thr Ala Cys Gln Gly Val Gly Gly
   1 5 10 15
   Pro Gly His Lys Ala Arg Val Leu Ala Glu
   20 25

### (2) ANGABEN ZU SEQ ID NO: 24:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 24:
   Gln Gly Val Gly Gly Pro Gly His Lys Ala Arg Val Leu Ala Glu
   1 5 10 15
   Ala Met Ser Gln Val Thr Asn Ser Ala Thr
   20 25

### (2) ANGABEN ZU SEQ ID NO: 25:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 25:
   Arg Val Leu Ala Glu Ala Met Ser Gln Val Thr Asn Ser Ala Thr
   1 5 10 15
   Ile Met Met Gln Arg Gly Asn Phe Arg Asn
   20 25

### (2) ANGABEN ZU SEQ ID NO: 26:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 26:
   Thr Asn Ser Ala Thr Ile Met Met Gln Arg Gly Asn Phe Arg Asn
    1               5                  10                  15
   Gln Lys Lys Thr Val Lys Cys Phe Asn Cys
                   20                  25

### (2) ANGABEN ZU SEQ ID NO: 27:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 27:
   Gly Asn Phe Arg Asn Gln Lys Lys Thr Val Lys Cys Phe Asn Cys
    1               5                  10                  15
   Gly Lys Glu Gly His Ile Ala Lys Asn Cys
                   20                  25

### (2) ANGABEN ZU SEQ ID NO: 28:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 28:
   Lys Cys Phe Asn Cys Gly Lys Glu Gly His Ile Ala Lys Asn Cys
    1               5                  10                  15
   Arg Ala Pro Arg Leu Lys Gly Cys Trp Lys
                   20                  25

### (2) ANGABEN ZU SEQ ID NO: 29:

(i) SEQUENZKENNZEICHEN:
   (A) LÄNGE: 25 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 29:
   Ile Ala Lys Asn Cys Arg Ala Pro Arg Leu Lys Gly Cys Trp Lys
    1               5                  10                  15
   Cys Gly Lys Glu Gly His Gln Met Lys Asp
                   20                  25

### (2) ANGABEN ZU SEQ ID NO: 30:

(i) SEQUENZKENNZEICHEN:
   (A) LÄNGE: 27 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 30:
   Lys Gly Cys Trp Lys Cys Gly Lys Glu Gly His Gln Met Lys Asp
    1               5                  10                  15
   Cys Thr Glu Arg Gln Ala Asn Phe Leu Gly Lys Ile
                   20                  25

### (2) ANGABEN ZU SEQ ID NO: 31:

(i) SEQUENZKENNZEICHEN:
   (A) LÄNGE: 15 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 31:
   Gln Ala Ile Ser Pro Arg Thr Leu Asn Ala Trp Val Lys Val Ile
   1               5                   10                  15

### (2) ANGABEN ZU SEQ ID NO: 32:

(i) SEQUENZKENNZEICHEN:
   (A) LÄNGE: 11 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 32:
   Ile Ser Pro Arg Thr Leu Asn Ala Trp Val Lys
   1               5                   10

### (2) ANGABEN ZU SEQ ID NO: 33:

(i) SEQUENZKENNZEICHEN:
   (A) LÄNGE: 15 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 33:
   Ile Asn Glu Glu Ala Ala Glu Trp Asp Arg Val His Pro Val His
   1 5 10 15

### (2) ANGABEN ZU SEQ ID NO: 34:

(i) SEQUENZKENNZEICHEN:
   (A) LÄNGE: 11 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 34:
   Glu Glu Ala Ala Glu Trp Asp Arg Val His Pro
   1 5 10

### (2) ANGABEN ZU SEQ ID NO: 35:

(i) SEQUENZKENNZEICHEN:
   (A) LÄNGE: 15 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 35:
   Ile Arg Gln Gly Pro Lys Glu Pro Phe Arg Asp Tyr Val Asp Arg
   1 5 10 15

### (2) ANGABEN ZU SEQ ID NO: 36:

(i) SEQUENZKENNZEICHEN:
   (A) LÄNGE: 11 Aminosäuren
   (B) ART: Aminosäure
   (C) STRANGFORM:
   (D) TOPOLOGIE: linear
(ii) ART DES MOLEKÜLS: Peptid
(xi) SEQUENZBESCHREIBUNG: SEQ ID NO: 36:
   Gln Gly Pro Lys Glu Pro Phe Arg Asp Tyr Val
    1               5                   10

## Beschreibung

Die Erfindung betrifft ein Verfahren zur Diagnose einer HIV-Infektion mittels eines Immunoassays durch den spezifischen Nachweis von HIV-Antigenen und HIV-Antikörpern.

AIDS (acquired immunodeficiency syndrome) ist eine erworbene ImmunschwächeKrankheit, die durch das HIV-Virus verursacht wird. Bisher sind als Erreger die Stämme HIV1 und HIV2 bekannt. Beide Stämme ähneln sich in Bezug auf Morphologie. Zelltropismus, Wechselwirkung mit dem CD4-Rezeptor von T-Zellen, den in vitro cytopathischen Effekt auf CD4-Zellen, die generelle genomische Struktur und die Fähigkeit, die Krankheit AIDS auszulösen (Clavel, 1987, AIDS 1, 135-140). Allerdings ist der immunologische Verwandschaftsgrad gering, so daß HIV1-spezifische Antikörper im allgemeinen keine Kreuzreaktion zu HIV2 zeigen. Neben den am weitesten verbreiteten HIV1 Gruppe M Subtypen ist noch ein weiterer HIV1-Subtyp, der Subtyp O bekannt (Myers et al, Los Alamos Datenbank. 1994; Sharp et al. AIDS Suppl. 8, S27-S42, 1994). Der Verwandtschaftsgrad zwischen HIV1-SubO und HIV1 ist wesentlich größer als zwischen HIV1-SubO und HIV2. Antikörper, die gegen HIV1 gerichtet sind. kreuzreagieren allerdings nur zum Teil mit dem entsprechenden Antigen von HIV1-SubO. Auch ein großer Teil der HIV1-SubO spezifischen Antikörper reagiert nicht mit HIV1 Gruppe M Antigenen.

Der Verlauf der HIV-Infektion läßt sich in mehrere diagnostisch relevante Phasen einteilen. In der Frühphase der Infektion können bereits HIV-Antigene, jedoch noch keine HIV-Antikörper nachgewiesen werden. In der Phase der Serokonversion können HIV-Antigene schwach positiv oder negativ, d.h. nicht nachweisbar sein. HIV-Antikörper der Klasse IgM sind in dieser Phase nachweisbar, HIV-Antikörper der Klasse IgG dagegen nicht oder nur schwach positiv. In der folgenden, symptomlosen Phase sind hauptsächlich HIV-Antikörper vom Typ IgG nachweisbar, während HIV-Antigen in der Regel nicht auftritt. Das gleiche gilt für den fortschreitenden Krankheitsverlauf in der klinischen Phase.

In der Spätphase der Erkrankung schließlich können HIV-Antikörper schwach positiv oder negativ werden, während die HIV-Antigene entweder negativ bleiben oder aufgrund der Zunahme der Viruslast, wenn das Immunsystem des Patienten zusammenbricht, wieder positiv nachgewiesen werden können. Bei diesen verschiedenen Phasen der Erkrankung, deren Verläufe sich patientenabhängig stark unterscheiden können, gibt es somit immer Zeitpunkte, an denen entweder Antigen- oder Antikörper-Nachweise falsch negative Werte ergeben können.

Zur Detektion von Infektionen mit HIV1, HIV2 oder HIV1-SubO werden häufig Antikörper-Tests (IgG und IgM) im Brückentest-Format, das beispielsweise in der EP-A-0 280 211 beschrieben ist, durchgeführt. Als Antigene werden hierfür im allgemeinen die Antigene des sogenannten Envelope-Bereiches (env), das sind gp160, gp120, gp41 für HIV1/HIV1-SubO und gp140, gp110, gp36 für HIV2, zusammen mit dem Antigen p24 (HIV1), aus dem der Viruskem aufgebaut ist, bzw. p26 (HIV2) eingesetzt. Die Antigene p24 bzw. p26 werden vom sogenannten gag-Bereich kodiert. Diese Tests ergeben dann ein positives Signal, wenn in der Probe Antikörper gegen die genannten Antigene vorhanden sind. In der Früh- und Spätphase der Erkrankung, wenn freies p24 bzw. p26-Antigen vorhanden ist, kommt es häufig vor, daß keine Antikörper nachweisbar sind, da entweder das Immunsystem des Patienten noch nicht genügend Antikörper gebildet hat oder so erschöpft ist, daß nicht mehr ausreichend Antikörper gebildet werden, um diese detektieren zu können.

Antigen-Tests zum Nachweis von p24-Antigen werden im allgemeinen getrennt von Antikörper-Tests durchgeführt. Nur in der Frühphase der Infektion und in der Endphase von AIDS ist der Antigen-Titer beim Patienten erhöht, so daß nur in diesen Phasen p24 sicher nachweisbar ist.

Nachteil der bisher bekannten Tests ist, daß keiner der Tests für sich genommen den gesamten diagnostisch relevanten Zeitraum der HIV-Infektion abdeckt.

Mit Hilfe von Kombitests können Antigene eines bestimmten Erregers und Antikörper gegen denselben Erreger gleichzeitig nachgewiesen werden. Ein solches Verfahren zur simultanen Bestimmung von Antigenen und Antikörpern wird in der DE 42 36 189 A1 offenbart. Ein Lösungsansatz zur lückenlosen diagnostischen Erfassung aller Phasen einer HIV-Infektion wird jedoch nicht beschrieben.

In der WO 93/21346 wird ein Kombitest zur simultanen Detektion von HIV 1 p24-Antigen, HIV1 gp41-Antikörpern und HIV2 gp36-Antikörpern (in beiden Fällen Antikörper gegen Proteine des env-Gens) mittels eines heterogenen Immunoassays beschrieben. Mit diesem Test werden jedoch HIV-positive Proben, die kein p24 oder keine Antikörper gegen die env-Proteine enthalten, nicht als positiv erfaßt. Nur mit gp41 können nicht alle Antikörperpositiven HIV1-Proben erkannt werden, da Proben weit verbreitet sind, die dadurch charakterisiert sind, daß sie im Western-Blot nur ein unvollständiges Bandenmuster aufweisen und keine Färbung der gp41-Bande zeigen.

Hashida et al (1996, J. Clin. Lab. Anal. 10, 213-219) beschreiben einen diagnostischen Test für den Nachweis von HIV1-Infektionen. Bei diesem Test werden gleichzeitig p24-Antigen. IgG-Antikörper gegen p17 (aus dem gag-Bereich) und IgG-Antikörper gegen Reverse Transkriptase (RT), die vom pol-Gen kodiert wird, nachgewiesen. Mit dieser Testführung ist allerdings nur der Nachweis von HIV1-Infektionen möglich. Außerdem werden Serokonversionsseren, die nur schwach affine IgM-Antikörper gegen die beiden oben verwendeten Antigene enthalten, nicht erfaßt. Außerdem werden Serokonversionsseren, die Antikörper gegen die env-Proteine enthalten, nicht erfaßt. Nach dem Abfall des HIV-Antigen-Titers in der Frühphase der Infektion treten oft nur IgM-Antikörper gegen das env-Protein gp41 auf. In diesem Fall zeigt der Test von Hashida et al. ein falsch negatives Ergebnis.

Es existiert im Stand der Technik bisher kein diagnostisches Testsystem, mit dem die simultane Detektion von HIV1, HIV1-SubO und HIV2 lückenlos für alle Infektionsstadien zuverlässig möglich ist.

Aufgabe war es daher, einen verbesserten Test auf HIV-Infektionen zu entwickeln, der es ermöglicht, als einzelner Test alle diagnostisch erfaßbaren Phasen der HIV-Infektion richtig, zuverlässig und lückenlos nachzuweisen. Der Test sollte die gleichzeitige Bestimmung von HIV1, HIV1-SubO und HIV2 ermöglichen.

Die Aufgabe wird gelöst durch das erfindungsgemäße Verfahren zur Diagnose einer HIV-Infektion mittels eines Immunoassays durch den spezifischen Nachweis von p24-Antigen von HIV1, HIV1-SubO und/oder p26-Antigen von HIV2, mindestens einem Antikörper gegen den env-Bereich von HIV1, HIV1-SubO und/oder HIV2 und mindestens einem Antikörper gegen den pol- und/oder gag-Bereich aus HIV1, HIV1-SubO und/oder HIV2, wobei der gag-Bereich nicht Sequenzen von p 24/p26 umfasst, und wobei die Bestimmung der einzelnen HIV-Antigene und HIV-Antikörper als einzelner Test simultan erfolgt. Mit Hilfe des erfindungsgemäßen Verfahrens ist es möglich, bereits beim Auftreten von nur einem der oben aufgeführten Analyten die HIV-Infektion sicher nachzuweisen.

Überraschenderweise hat sich gezeigt, daß durch den Nachweis von Antikörpern gegen ein Genprodukt insbesondere des pol-Bereichs von HIV in Kombination mit dem Nachweis von p24-Antigen von HIV1, HIV1-SubO und/oder p26-Antigen von HIV2 und dem Nachweis von mindestens einem Antikörper gegen den HIV-env-Bereich die bisher zu beklagende diagnostische Lücke geschlossen werden kann. Überraschenderweise hat sich ein Verfahren zur Detektion einer HIV-Infektion als besonders geeignet erwiesen, in dem Antikörper gegen die Reverse Transkriptase (RT) von HIV nachgewiesen werden, in Kombination mit dem Nachweis von p24-Antigen von HIV1, HIV1-SubO und/oder p26-Antigen von HIV2 und dem Nachweis von mindestens einem Antikörper gegen den HIV-env-Bereich.

Der Nachweis der HIV-Infektion durch den erfindungsgemäßen Kombitest mittels Bestimmung der einzelnen genannten Parameter erfolgt simultan als einzelner Test. Die Bezeichnung "Kombitest" bedeutet, daß gleichzeitig HIV-Antigene und Antikörper, die gegen HIV-Antigene gerichtet sind, nachgewiesen werden können. Mit Hilfe dieses Testes können alle HIV-Infektionsstadien sicher erfaßt werden. Durch die erfindungsgemäße Auswahl von Test-Antigenen bzw.-Antikörpern und den dazugehörigen spezifisch bindenden Rezeptoren ist es möglich, das erfindungsgemäße Verfahren durchzuführen. Zudem ist der Nachweis von HIV1, HIV1-SubO und HIV2 mit einem einzigen Test möglich. Darüberhinaus ist durch geeignete Wahl von Rezeptoren eine breite Erkennung von HIV1-Subtypen der HIV1-Gruppe M möglich. Der Test beruht bevorzugt auf dem Prinzip des heterogenen Immunoassays. Jedoch sind auch homogene Testführungen wie beispielsweise turbidimetrische Tests, bei denen keine Trennung von fester und flüssiger Phase erfolgt, denkbar.

Erfindungsgemäß werden im Nachweisverfahren als Rezeptoren R1 und R2 solche eingesetzt, die das zu bestimmende HIV1-p24- und/oder HIV2-p26-Antigen spezifisch binden. Als Rezeptoren R3 und R4 werden ein oder mehrere Antigene aus dem env-Bereich von HIV1, HIV2 oder HIV1-SubO verwendet (gp160, gp120, gp41 für HIV1/HIV1-SubO und gp140, gp110, gp36 für HIV2). Bevorzugt werden als Rezeptoren R3 und R4 gp41 und/oder gp36 oder Fragmente davon verwendet. Als Rezeptoren R5 und R6 werden ein oder mehrere Antigene aus dem pol- oder gag-Bereich von HIV1, HIV2, oder HIV1-SubO verwendet, wobei dies nicht Epitope oder Sequenzen von p24 oder p26 sein dürfen, die mit den Rezeptoren R1 oder R2 reagieren können. Bevorzugt werden als R5 und R6 Antigene aus dem pol-Bereich von HIV1, HIV2, oder HIV1-SubO eingesetzt. Besonders bevorzugt wird als Rezeptor R5 und R6 die Reverse Transkriptase (RT) verwendet.

Durch die erfindungsgemäße Kombination von Rezeptoren ist es möglich, alle Stadien der HIV-Infektion sicher zu detektieren. Es werden dadurch sowohl HIV-spezifische Analyten nachgewiesen, die nur in der Früh- oder Spätphase der Infektion vorhanden sind (z.B. p24-bzw. p26-Antigen), als auch Analyten, die in der asymptomatischen Phase auftauchen und über einen längeren Zeitraum detektierbar sind, wie beispielsweise Antikörper gegen die env-Genprodukte gp160, gp120 oder gp41 und Antikörper gegen die pol-Genprodukte Reverse Transkriptase, Integrase oder Protease oder Antikörper gegen die gag-Genprodukte p17 oder p15.

Durch geeignete Wahl der Rezeptoren können, falls gewünscht, mit einem einzigen Test Infektionen mit HIV1, HIV2 und HIV1-SubO einschließlich aller HIV1-Subtypen der Gruppe M nachgewiesen werden. Für eine solche Testführung können als Rezeptoren R1 und R2 solche ausgewählt werden, die spezifisch das p24-Antigen von HIV1 binden. R1 und R2 werden in diesem Fall so gewählt, daß sowohl HIV1-p24 als auch HIV1-SubO-p24 erkannt und gebunden wird. Dabei kann es notwendig sein, mehrere verschiedene Rezeptoren für R1 und R2 zu verwenden, um alle HIV1 Gruppe M Subtypen und den HIV1 Subtyp O zu detektieren. Zusätzlich werden beim gleichzeitigen Nachweis von HIV1, HIV2 und HIV1-SubO entweder entsprechende kreuzreagierende oder weitere Rezeptoren R1 und R2 eingesetzt, die spezifisch auch das p26-Antigen von HIV2 erkennen. Die Anzahl der insgesamt zu verwendenden Rezeptoren R1 und R2 ist abhängig von der Kreuzreaktivität bzw. Homologie der gewählten Epitope. Durch günstige Auswahl ist es möglich, die Anzahl der verwendeten Rezeptoren zu reduzieren. Als Rezeptoren R3 und R4 können solche eingesetzt werden, die Antikörper gegen env-Genprodukte von HIV1 und HIV1-SubO spezifisch binden. Besonders geeignet ist hierbei das gp41-Antigen (HIV1 bzw. HIV1-SubO) oder Fragmente bzw. Epitope hiervon. Als weitere Rezeptoren R3 und R4 werden solche Rezeptoren eingesetzt, die Antikörper gegen env-Genprodukte von HIV2 spezifisch erkennen. Als besonders geeignet erweist sich hierbei das gp36-Antigen oder Fragmente bzw. Epitope hiervon. Als Rezeptoren R5 und R6 können solche Rezeptoren ausgewählt werden, die Antikörper gegen gag- oder pol-Genprodukte von HIV1 und HIV1-SubO spezifisch erkennen - mit Ausnahme der Epitope von p24, die mit den Rezeptoren R1 oder R2 reagieren können. Zusätzlich können für die HIV2-Erkennung weitere Rezeptoren R5 und R6 verwendet werden, die Antikörper gegen gag- oder pol-Genprodukte von HIV2 spezifisch binden. Bevorzugt für alle Rezeptoren R5 und R6 sind solche, die Antikörper gegen die pol-Genprodukte von HIV1 und HIV1-SubO bzw. von HIV2 erkennen. Von den Rezeptoren R5 und R6 gegen die drei möglichen pol-Genprodukte Reverse Transkriptase, Integrase und Protease werden besonders bevorzugt solche Rezeptoren verwendet, die Antikörper gegen die Reverse Transkriptase erkennen.

Gegenstand der Erfindung ist auch das oben beschriebene Verfahren zum Nachweis einer HIV-Infektion, wobei die Rezeptoren R1 und R2 jeweils als einzelne Rezeptoren oder in Form von Gemischen verschiedener Rezeptoren eingesetzt werden. Bei Verwendung von Gemischen erkennen die Rezeptoren bevorzugt unterschiedliche Epitope des p24 bzw. p26.

Der Einsatz der Rezeptorgemische dient dazu, die Erkennung verschiedener HIV-Subtypen - im Falle des p24 Antigens die Erkennung von HIV1 Gruppe M Subtypen und HIV1-SubO - mit einer einzigen Testzusammensetzung zu gewährleisten.

Erfindungsgemäß können mit dem Nachweisverfahren auch Infektionen mit dem HIV1-Subtyp O detektiert werden.

Außerdem können mit dem Nachweisverfahren erfindungsgemäß auch Infektionen mit den HIV1-Subtypen der Gruppe M nachgewiesen werden.

Erfindungsgemäß können daher auch die Rezeptoren R3, R4, R5 und R6 jeweils in Form von Gemischen verschiedener Rezeptoren eingesetzt werden, um zu gewährleisten, daß HIV1-, HIV1-SubO- und HIV2-positive Proben in einem Testansatz sicher erkannt werden. Es muß jedoch auch immer gewährleistet sein, daß sich die verschiedenen Rezeptoren gegenseitig nicht wesentlich in ihrer Bindung um das nachzuweisende Antigen bzw. den nachzuweisenden Antikörper behindern. Bevorzugt werden als Rezeptoren R3 und R4 solche eingesetzt. die jeweils die gleichen Epitope präsentieren. Dadurch wird gewährleistet, daß die antigenbindenden Teile des nachzuweisenden Antikörpers die beiden Rezeptoren R3 und R4 verbrückt. Je nach Anforderung können aber auch Kombinationen verschiedener Epitope verwendet werden. Beispielsweise könnte als R3 rekombinantes gp41 und als R4 Peptide, die vom gp41 abgeleitet sind, oder Polyhaptene - wie sie in der WO 96/03652 beschrieben sind - eingesetzt werden. Das gleiche gilt für die Rezeptoren R5 und R6. Je nach Anforderung können Kombinationen verschiedener Epitope verwendet werden. Jedoch werden auch hier bevorzugt solche Rezeptoren eingesetzt, die die gleichen Epitope darstellen.

Das erfindungsgemäße Verfahren wird im allgemeinen als Naßtest durchgeführt. Neben den sogenannten Naßtesten, bei denen die Testreagenzien in flüssiger Phase vorliegen, können auch alle gängigen Trockentestformate, die zum Nachweis von Proteinen bzw. Antikörpern geeignet sind, verwendet werden. Bei diesen Trockentests oder Teststreifen, wie sie beispielsweise in der EP-A-0 186 799 beschrieben sind. sind sämtliche Testkomponenten auf einem Träger aufgebracht.

Beim erfindungsgemäßen Verfahren, das bevorzugt als heterogener Immunoassay durchgeführt wird, erfolgt der Nachweis der Antigene durch die Rezeptoren R1 und R2 nach dem Sandwich-Prinzip. Das nachzuweisende Antigen (hier: p24 bzw. p26) wird von R1 und R2 in Form eines Sandwiches von zwei Seiten gebunden. Die Bindung der Antikörper durch die Rezeptoren R3, R4, R5 und R6 erfolgt nach dem Prinzip des Brückentests. Der zu detektierende Antikörper verbrückt die Rezeptoren R3 und R4 miteinander. Das gleiche gilt für die Rezeptoren R5 und R6. Beide Testführungen, der Sandwich- und der Brückentest, sind gleichzeitig im gleichen Ansatz durchführbar, ohne daß die beiden Prinzipien sich gegenseitig stören. Es ist daher auch möglich, alle Rezeptoren mit der Probe zusammen zu inkubieren und das Verfahren in wenigen Stufen durchzuführen. Lediglich vor der Nachweisreaktion ist ein Wasch-Schritt zur Abtrennung der ungebundenen Rezeptoren und Probenbestandteile von Vorteil, aber nicht unbedingt notwendig. Die Rezeptoren R1, R3 und R5, die mit der Festphase bindefähig sind, können in flüssiger Phase oder bereits an die Festphase gebunden vorliegen. Bevorzugt werden alle Rezeptoren R1 bis R6 zusammen inkubiert. Dabei kann die Festphase, an die R1, R3 und R5 binden können, bereits anwesend sein oder erst später zugegeben werden oder per Diffusion oder Transfer erst später erreicht werden.

Bei der Inkubation bildet sich der Sandwich zwischen Festphase•R1•p24(p26)•R2, die Brücke zwischen Festphase•R3•HIV-Anti-env-Antikörper•R4 und die Brücke zwischen Festphase•R5•HIV-Anti-gag/polAntikörper•R6 aus. Anschließend wird im Falle des heterogenen Immunoassays die feste von der flüssigen Phase getrennt, gegebenenfalls die feste Phase gewaschen und die Markierung von R2, R4 und R6 bestimmt. Die Markierung wird zumeist an der festen Phase gemessen, sie kann jedoch auch in der flüssigen Phase bestimmt werden.

Liegen einer oder mehrere der Rezeptoren R1, R3 und R5 bereits in festphasengebundener Form vor, so werden die Probe und die entsprechenden Rezeptoren, d.h. R2, R4 und R6 zu den festphasengebundenen Rezeptoren R1, R3 und R5 dazugegeben und zusammen inkubiert. Es ist aber auch möglich, die Probe zuerst mit den Rezeptoren R1, R3 und R5 in An- oder Abwesenheit der Festphase zusammenzubringen und erst in einem nachfolgenden Schritt die Rezeptoren R2, R4 und R6 zuzugeben.

Ebenfalls offenbart ist ein Verfahren zum Nachweis von Antikörpern gegen HIV mittels eines Immunoassays, das dadurch gekennzeichnet ist, daß die Rezeptoren R3, R4, R5 und R6 eingesetzt werden. Die Rezeptoren R1 und R2 kommen bei diesem reinen Antikörpernachweis nicht zum Einsatz. Als Rezeptoren R3 und R4 werden wie im zuvor beschriebenen Kombitest ein oder mehrere Antigene aus dem env-Bereich von HIV1, HIV2 oder HIV1-SubO verwendet (gp160, gp120, gp41 für HIV1/HIV1-SubO und gp140, gp110. gp36 für HIV2). Bevorzugt werden als Rezeptoren R3 und R4 gp41 und/oder gp36 oder Fragmente davon verwendet. Als Rezeptoren R5 und R6 werden wie im zuvor beschriebenen Kombitest ein oder mehrere Antigene aus dem pol- oder gag-Bereich von HIV1, HIV2, oder HIV1-SubO verwendet. Bevorzugt werden als R5 und R6 Antigene aus dem pol-Bereich von HIV1, HIV2, oder HIV1-SubO eingesetzt. Besonders bevorzugt wird als Rezeptor R5 und R6 die Reverse Transkriptase (RT) verwendet.

Die Durchführung des Antikörpemachweises, die Testformate sowie die Eigenschaften der verwendeten Rezeptoren sind analog zum Kombitest, so daß sie hier nicht gesondert aufgeführt werden. Die im folgenden gemachten Ausführungen zu den Rezeptoren R1 und R2 gelten für den weiter oben beschriebenen kombinierten Nachweis (Kombitest) von HIV-Antikörpern und HIV-Antigenen. Die Ausführungen für die Rezeptoren R3, R4, R5 und R6 gelten sowohl für den Kombitest als auch für das Verfahren zum Nachweis von HIV-Antikörpern.

Wesentlicher Bestandteil von Rezeptor R1 ist ein Antikörper, der spezifisch das p24-Antigen von HIV1 und gegebenenfalls von HIV1-SubO oder das p26-Antigen von HIV2 bindet. Es können Antikörper aller Subklassen, die mit dem p24 oder dem p26 spezifisch bindefähig sind. verwendet werden. Statt der vollständigen Antikörper können selbstverständlich auch deren Fragmente, wie Fab-, Fab'- oder F(ab')₂-Fragmente, verwendet werden. Die Antikörper können polyklonal sein. sofern sie keine Kreuzreaktivität zu übrigen Testbestandteilen zeigen. Da an die Spezifität der Anti-p24-bzw. Anti-p26-Antikörper bezüglich der HIV-Subtypenerkennung hohe Anforderungen zu stellen sind, werden jedoch bevorzugt monoklonale Antikörper eingesetzt. Gegenstand der Erfindung sind daher auch monoklonale Antikörper gegen das p24-Antigen. Die Eigenschaften dieser Antikörper werden in einem späteren Abschnitt näher erläutert.

R1 kann entweder direkt an die Festphase gebunden sein, oder die Bindung an die Festphase erfolgt indirekt über ein spezifisches Bindungssystem. Die direkte Bindung von R1 an die Festphase erfolgt nach dem Fachmann bekannten Methoden. Wird die Bindung indirekt über ein spezifisches Bindungssystem durchgeführt, so ist R1 ein Konjugat, das aus einem Antikörper gegen p24 bzw. gegen p26 und einem Reaktionspartner eines spezifischen Bindungssystems besteht. Unter einem spezifischen Bindungssystem werden hier zwei Partner verstanden, die spezifisch miteinander reagieren können. Das Bindungsvermögen kann dabei auf einer immunologischen Reaktion oder auf einer anderen spezifischen Reaktion beruhen. Bevorzugt wird als spezifisches Bindungssystem eine Kombination von Biotin und Avidin oder Biotin und Streptavidin verwendet. Weitere bevorzugte Kombinationen sind Biotin und Antibiotin, Hapten und Anti-Hapten, Fc-Fragment eines Antikörpers und Antikörper gegen dieses Fc-Fragment oder Kohlenhydrat und Lectin. Einer der Reaktionspartner dieses spezifisch bindefähigen Paares ist dann Teil des Konjugates, das den Rezeptor R1 bildet.
Der andere Reaktionspartner des spezifischen Bindungssystems für R1 liegt als Beschichtung der festen Phase vor. Die Bindung des anderen Reaktionspartners des spezifischen Bindungssystems an ein unlösliches Trägermaterial kann nach den üblichen, dem Fachmann bekannten Methoden vorgenommen werden. Hierbei ist sowohl eine kovalente als auch eine adsorptive Bindung geeignet. Als feste Phase geeignet sind Reagenzgläschen oder Mikrotiterplatten aus Polystyrol oder ähnlichen Kunststoffen, die an der Innenoberfläche mit einem Reaktionspartner des spezifischen Bindungssystems beschichtet sind. Weiterhin geeignet und besonders bevorzugt sind teilchenförmige Substanzen, wie beispielsweise Latexpartikel, magnetische Partikel, Molekularsiebmaterialien, Glaskörperchen, Kunststoffschläuche und dergleichen. Auch poröse, schichtförmige Träger wie Papier können als Träger verwendet werden. Besonders bevorzugt werden magnetische Kügelchen (sogenannte Beads) verwendet, die wiederum mit dem entsprechenden Bindepartner des oben beschriebenen spezifischen Bindesystems beschichtet sind. Diese Mikropartikel können dann nach Ablauf der Testreaktion für die Durchführung der Nachweisreaktion beispielsweise durch Filtration, Zentrifugation oder im Falle der magnetischen Partikel durch einen Magneten von der flüssigen Phase getrennt werden.

Der Rezeptor R2 besteht aus einem Antikörper, der spezifisch das p24-Antigen von HIV1 und gegebenenfalls von HIV1-SubO oder das p26-Antigen von HIV2 bindet, und einer Markierung. Es können - wie beim Rezeptor R1 - Antikörper aller Subklassen, die mit dem p24 oder dem p26 spezifisch bindefähig sind, verwendet werden. Statt der vollständigen Antikörper können auch hier deren Fragmente, wie Fab-, Fab'- oder F(ab')₂-Fragmente, verwendet werden. Die Antikörper können polyklonal oder monoklonal sein. Bevorzugt werden wie bei R1 monoklonale Antikörper eingesetzt. Besonders bevorzugt werden auch bei R2 die in einem späteren Abschnitt beschriebenen erfindungsgemäßen monoklonalen Antikörper gegen das p24-Antigen verwendet. Entscheidend ist, daß die in R1 und R2 eingesetzten Antikörper mindestens 2 verschiedene Epitope des p24- bzw. des p26-Antigens erkennen. Beide Rezeptoren R1 und R2 müssen gleichzeitig spezifisch an p24 oder p26 binden können. um den Sandwich auszubilden. Die von den Antikörper-Bestandteilen in R1 und R2 erkannten Epitope müssen sich also räumlich voneinander getrennt befinden. Das heißt, daß die von R1 und R2 erkannten Epitope zwar überlappen dürfen, jedoch muß gewährleistet sein, daß beide Rezeptoren gleichzeitig an das p24 bzw. p26-Epitop binden können. Bedingung ist immer, daß sich die beiden Rezeptoren in ihrem Bindungsverhalten an p24 bzw. p26 gegenseitig nicht wesentlich behindern.

Beim Einsatz von Rezeptorgemischen für R1 und R2 ist es auch möglich, die gleichen Rezeptoren sowohl für R1 als auch für R2 zu verwenden. Eine bestimmte Anzahl an R1-Rezeptoren wäre dann mit der Festphase bindefähig, die verbleibenden R1-Rezeptoren wären nicht mit der Festphase bindefähig und würden stattdessen die Markierung tragen. Das gleiche gilt dann für R2. Der Sandwich kann sich dann sowohl in der Orientierung Festphasee•R1•p24•R2 als auch in der Orientierung Festphase•R1(R2-Epitop)•p24•R2(R1-Epitop) ausbilden.

Ein weiterer Bestandteil des Rezeptors R2 ist die Markierung, Bevorzugt wird als Markierung eine direkt nachweisbare Substanz, beispielsweise eine chemilumineszierende, elektrochemilumineszierende, fluoreszierende oder radioaktive Substanz oder ein Metallsol-, Latex- oder Goldpartikel eingesetzt. Weiterhin bevorzugt als Markierung sind Enzyme oder andere Moleküle wie Haptene (z.B. Digoxigenin) oder Fluoreszenzfarbstoffe, beispielsweise Fluorescein. Eine besonders bevorzugte Markierung sind elektrochemilumineszierende Metallchelate wie sie in der WO 96/03651 beschrieben sind. Als Metallchelate werden bevorzugt Rutheniumkomplexe eingesetzt, die ebenfalls in der WO 96/03651 offenbart sind. Die Verfahren zur Markierung sind dem Fachmann geläufig und bedürfen hier keiner weiteren Erklärung. Der Nachweis der Markierung erfolgt in an sich bekannter Weise direkt durch Messung der chemilumineszierenden, fluoreszierenden oder radioaktiven Substanz oder des Metallsol-, Latex- oder Goldpartikels oder durch Messung des durch das Enzym umgesetzten Substrats.

Der Nachweis der Markierung kann auch auf indirekte Weise erfolgen. Dabei bindet ein weiterer Rezeptor, der selbst wiederum mit einer signalerzeugenden Gruppe gekoppelt ist. spezifisch an die Markierung von R2, beispielsweise ein Hapten wie Digoxigenin. Die Herstellung von haptenmarkierten und insbesondere digoxigeninmarkierten Rezeptoren wird in der WO 96/03423 beschrieben.

Der Nachweis der signalerzeugenden Gruppe, beispielsweise eine elektrochemilumineszierende, fluoreszierende oder radioaktive Substanz oder ein Enzym oder Goldpartikel, erfolgt nach dem Fachmann geläufigen Methoden. Als weiterer Rezeptor kann beispielsweise ein Antikörper oder ein Antikörperfragment eingesetzt werden, der spezifisch an die Markierung von R2 bindet. Wird dieser indirekte Nachweis der Markierung verwendet, so ist die Markierung von R2 bevorzugt Digoxigenin oder ein anderes Hapten. und der Nachweis erfolgt über einen Antikörper, der gegen Digoxigenin oder gegen das Hapten gerichtet ist und mit Peroxidase oder einer anderen Markierung wie oben beschrieben markiert ist.

Bevorzugt werden als Bestandteile der Rezeptoren R1 und R2 monoklonale Antikörper oder deren Fragmente eingesetzt. Gegenstand der vorliegenden Erfindung sind daher auch monoklonale Antikörper, die das p24-Antigen von HIV1 spezifisch mit ausreichend hoher Affinität zur Ausbildung eines Sandwichs binden. Die monoklonalen Antikörper können dadurch in allen dem Fachmann geläufigen Tests zum Nachweis eines Proteins wie beispielsweise im Sandwich-Test verwendet werden.

Die monoklonalen Antikörper können allen möglichen Immunglobulinklassen (Ig) angehören. Bevorzugt gehören die monoklonalen Antikörper der IgG1-Klasse an. Die Kopplung von weiteren Komponenten wie beispielsweise Markierungen wie Enzyme oder Haptene oder Bindepartner, die zur Bindung des Antikörpers an eine Festphase bei heterogenen Immunoassays benötigt werden, kann bevorzugt an IgG1-Antikörper erfolgen. Auch die Herstellung von Antikörper-Fragmenten wie beispielsweise F(ab')₂-, Fab'- oder Fab-Fragmenten ist bei der IgG1-Klasse unproblematisch.

Erfindungsgemäß werden unter der Bezeichnung "monoklonaler Antikörper" sowohl der vollständige Antikörper als auch alle bei Immuntests und sonstigen Verwendungen gängigen Fragmente davon, wie F(ab')₂-, Fab'- oder Fab-Fragmente, verstanden. Umfaßt sind auch solche Antikörper, die durch Veränderung der monoklonalen Antikörper hergestellt wurden, solange die Antigenbindeeigenschaft nicht entscheidend beeinflußt wurde. Beispielsweise können durch gentechnologische Maßnahmen Teile der normalerweise in Mäusen hergestellten monoklonalen Antikörper durch entsprechende humane Antikörpersequenzen ersetzt werden, um unspezifische Bindungen im Immunoassay zu minimieren. Verfahren zur Herstellung solcher chimärer monoklonaler Antikörper sind dem Fachmann beispielsweise aus Antibody Engineering, J. Mc Cafferty, H.R. Hoogenboom und D.J. Chiswell, The Practical Approach Series, Series Editor: B.D. Harnes, Oxford University Press, 1996, bekannt.

Entscheidend für die Eignung der Antikörper sind die Epitope des p24, die von den Antikörpern erkannt werden, da diese für die Erkennung aller Subtypen entscheidend sind. Für die p24-Epitope wurde die Strukturinformation aus Gitti et al (Science 1996, Vol 273, S. 231-235) zugrundegelegt. Geeignet sind demnach auch anders erzeugte Antikörper, die die gleichen Epitope wie die im folgenden beschriebenen Antikörper oder zu diesen überlappende Epitope erkennen. Die Epitope lassen sich beispielsweise durch die Pepscan-Methode charakterisieren. Im Beispiel 4 der vorliegenden Anmeldung ist die genaue Vorgehensweise bei der Epitop-Kartierung der erfindungsgemäßen Antikörper beschrieben. Bevorzugt wird der Epitopbereich des p24, an den die Antikörper binden, ausgewählt aus der Gruppe der folgenden Aminosäuresequenzen. Diese Sequenzen sind auch im Sequenzprotokoll unter SEQ ID NO 1-36 angegeben.

| **Sequenz** | **Peptid** | **SEQ ID NO** |
|---|---|---|
| DKGNSSQVSQNYPIVQNLQGQMVHQ | 1 | 1 |
| NYPIVQNLQGQMVHQAISPRTLNAW | 2 | 2 |
| **QMVHQAISPRTLNAWVKVIEEKAFS** | **3** | **3** |
| TLNAWVKVIEEKAFSPEVIPMFSAL | 4 | 4 |
| EKAFSPEVIPMFSALSEGATPQDLN | 5 | 5 |
| MFSALSEGATPQDLNTMLNTVGGHQ | 6 | 6 |
| PQDLNTMLNTVGGHQAAMQMLKETI | 7 | 7 |
| VGGHQAAMQMLKETINEEAAEWDRV | 8 | 8 |
| **LKETINEEAAEWDRVHPVHAGPIAP** | **9** | **9** |
| EWDRVHPVHAGPIAPGQMREPRGSD | 10 | 10 |
| GPIAPGQMREPRGSDIAGTTSTLQE | 11 | 11 |
| PRGSDIAGTTSTLQEQIGWMTNNPP | 12 | 12 |
| STLQEQIGWMTNNPPIPVGEIYKRW | 13 | 13 |
| TNNPPIPVGEIYKRWIILGLNKIVR | 14 | 14 |
| IYKRWIILGLNKIVRMYSPVSILDI | 15 | 15 |
| NKIVRMYSPVSILDIRQGPKEPFRD | 16 | 16 |
| **SILDIRQGPKEPFRDYVDRFYKTLR** | **17** | **17** |
| EPFRDYVDRFYKTLRAEQASQEVKN | 18 | 18 |
| YKTLRAEQASQEVKNWMTETLLVQN | 19 | 19 |
| QEVKNWMTETLLVQNANPDCKTILK | 20 | 20 |
| LLVQNANPDCKTILKALGPAATLEE | 21 | 21 |
| KTILKALGPAATLEEMMTACQGVGG | 22 | 22 |
| ATLEEMMTACQGVGGPGHKARVLAE | 23 | 23 |
| QGVGGPGHKARVLAEAMSQVTNSAT | 24 | 24 |
| RVLAEAMSQVTNSATIMMQRGNFRN | 25 | 25 |
| TNSATIMMQRGNFRNQKKTVKCFNC | 26 | 26 |
| GNFRNQKKTVKCFNCGKEGHIAKNC | 27 | 27 |
| KCFNCGKEGHIAKNCRAPRLKGCWK | 28 | 28 |
| IAKNCRAPRLKGCWKCGKEGHQMKD | 29 | 29 |
| KGCWKCGKEGHQMKDCTERQANFLGKI | 30 | 30 |
| **QAISPRTLNAWVKVI** | **3A** | **31** |
| ISPRTLNAWVK | 3B | 32 |
| INEEAAEWDRVHPVH | 9A | 33 |
| **EEAAEWDRVHP** | **9B** | **34** |
| IRQGPKEPFRDYVDR | 17A | 35 |
| QGPKEPFRDYV | 17B | 36 |

Offenbart der Erfindung sind monoklonale Antikörper gegen HIV1 p24-Antigen, die die p24-Epitope gemäß SEQ ID NO: 1-36 spezifisch binden.

Weiterhin offenbart sind monoklonale Antikörper gegen HIV1 p24-Antigen, die die p24-Epitope gemäß SEQ ID NO: 3, 9, 17, 31 oder 34 spezifisch binden. Diese Sequenzen sind in der obigen Aufstellung fett hervorgehoben.

Die erfindungsgemäßen monoklonalen Antikörper werden aus den Zellinien MAK<p24>M-6A9/5, MAK<p24>M-4B1/1, MAK<p24>M-6D9/4, MAK<p24>M-2E7/3 oder MAK<p24>M6A9/5 hergestellt. Die monoklonalen Antikörper MAK<p24>M-4B1/1 (Hinterlegungsnummer DSM ACC2299). MAK<p24>M-6D9/4 (Hinterlegungsnummer DSM ACC2300) und MAK<p24>M-2E7/3 (Hinterlegungsnummer DSM ACC2301), wurden am 26.02.1997 bei der DSMZ (Deutsche Sammlung von Mikroorganismen und Zellkulturen GmbH. Mascheroder Weg 1b, D-38124 Braunschweig) hinterlegt. Der monoklonale Antikörper MAK<p24>M6A9/5 wurde am 11.06.1997 bei der DSMZ unter der Hinterlegungsnummer DSM ACC2310 hinterlegt.

Mit dem in Beispiel 4 beschriebenen Epitopkartierungsverfahren wurde festgestellt. daß die Antikörper, die von der Zellinie MAK<p24>M-6A9/5 produziert werden, kein sequentielles Epitop erkennen, sondern an ein Konformationsepitop des p24 binden.

Mit dem gleichen Verfahren wurde festgestellt, daß die Antikörper, die von der Zellinie MAK<p24>M-2E7/3 (DSM ACC2301) produziert werden, an das Epitop binden, das einem Peptid gemäß SEQ ID NO: 9 entspricht (p24-Strukturinformation gemäß Gitti et al 1996, Science 273, 231-235). Es konnte außerdem gezeigt werden, daß das eigentliche Epitop noch kleiner ist. Zur spezifischen Bindung des MAK<p24>M-2E7/3 an p24 ist ein nur 11 Aminosäuren umfassendes Epitop gemäß SEQ ID NO: 34 ausreichend.

Mit dem gleichen Verfahren wurde festgestellt. daß die Antikörper, die von der Zellinie MAK<p24>M-6D9/4 (DSM ACC2300) produziert werden, an das Epitop binden, das einem Peptid gemäß SEQ ID NO: 3 entspricht. Außerdem konnte gezeigt werden, daß das für die spezifische Bindung erforderliche Epitop noch kleiner ist: Zur spezifischen Bindung des MAK<p24>M-6D9/4 an p24 ist ein 15 Aminosäuren umfassendes Epitop gemäß SEQ ID NO: 31 ausreichend.

Mit dem gleichen Verfahren wurde festgestellt, daß die Antikörper, die von der Zellinie MAK<p24>M-4B1/1 (DSM ACC2299) produziert werden, an das Epitop binden, das einem Peptid gemäß SEQ ID NO: 17 entspricht.

Die von den Zellinien MAK<p24>M-4B1/1 und MAK<p24>M-6D9/4 produzierten Antikörper binden nicht nur die zuvor charakterisierten p24-Epitope von HIV1, sondern auch von HIV1-SubO. Für die gleichzeitige Erkennung des p24-Antigens von HIV1 und HIV1-SubtypO sind daher besonders die von diesen Zellinien produzierten Antikörper geeignet. In Beispiel 5 ist die Austestung der erfindungsgemäßen Antikörper in Bezug auf die p24-Subtypenerkennung dargestellt. Von den beiden Antikörpern wird MAK<p24>M-4B1/1 bevorzugt auf der Festphasen-Seite, d.h. als Rezeptor R1 eingesetzt, und MAK<p24>M-6D9/4 wird bevorzugt als Rezeptor R2 verwendet.

Besonders bevorzugt werden für eine effektive gleichzeitige Erkennung von HIV1 und HIV1-SubO die Antikörper MAK<p24> 4B1/1 und MAK<p24>6A9 als R1 und MAK<p24>2E7/3 und MAK <p24>6D9/4 als R2 eingesetzt, da so die umfassendste Erkennung verschiedener HIV1 und HIV1-Subtyp O Virusisolate erreicht wird (siehe Beispiel 5).

Geeignet zur Verwendung im erfindungsgemässen Verfahren sind weiterhin Antikörper, bevorzugt monoklonale Antikörper, die in äquivalenter Weise an p24 binden, wie die monoklonalen Antikörper MAK<p24>M-6A9/5 (DSM ACC2310). MAK<p24>M-4B1/1 (DSM ACC2299). MAK<p24>M-6D9/4 (DSM ACC2300) oder MAK<p24>M-2E7/3 (DSM ACC2301). Unter "in äquivalenter Weise binden" wird verstanden, daß diese Antikörper mit gleich hoher oder vergleichbar hoher Affinität an p24 wie die hinterlegten monoklonalen Antikörper binden. Dies kann beispielsweise durch entsprechende Versuche am BIAcore® oder auch mit dem 2-Schritt-Sandwich-ELISA gemäß Beispiel 4 festgestellt werden.

Geeignet zur Verwendung im erfindungsgemässen Verfahren sind weiterhin Antikörper, bevorzugt monoklonale Antikörper, die an die gleichen Epitope an p24 binden, wie die hinterlegten Antikörper MAK<p24>M-6A9/5 (DSM ACC2310), MAK<p24>M-4B1/1 (DSM ACC2299), MAK<p24>M-6D9/4 (DSM ACC2300) oder MAK<p24>M-2E7/3 (DSM ACC2301). Dies kann durch Bestimmung der Kreuzreaktivität der Antikörper, beispielsweise durch Kompetitionsexperimente im ELISA oder mit dem BIAcore^{R}-System festgestellt werden. Bevorzugt besitzen diese Antikörper ebenfalls eine hohe Affinität zu p24.

Ein weiterer Gegenstand der Erfindung ist die Verwendung mindestens von einem der in den oberen Abschnitten beschriebenen hinterlegten Antikörpern in einem diagnostischen Test zum Nachweis einer HIV-Infektion. Ebenfalls Gegenstand der Erfindung ist die Verwendung der beschriebenen Antikörper in dem weiter oben erläuterten erfindungsgemäßen Kombitest zum Nachweis einer HIV-Infektion.

Die monoklonalen Antikörper können in an sich bekannter Weise durch Immunisierung mit isoliertem p24 (aus humanem Gewebe isoliert oder rekombinant) in geeigneten Versuchstieren wie beispielsweise Mäuse, Ratten, Kaninchen und anschließender Fusion der Milzzellen der immunisierten Tiere mit Myelomzellen hergestellt werden. Neben Milzzellen als Lymphocyten-Quelle können auch periphere Blut-Lymphocyten (PBL) oder Lymphknoten-Zellen von immunisierten Tieren (bevorzugt: Maus oder Ratte) verwendet werden. Anstelle von isoliertem p24 können zur Immunisierung auch synthetische, von p24 abgeleitete Peptide, die alleine oder an Träger gekoppelt verwendet werden, zur Erzeugung der gewünschten Antikörper verwendet werden.

Alternativ können auch Lymphocyten von humanen Spendern. die Antikörper gegen p24 entwickelt haben, immortalisiert werden. Solche Anti-p24-Antikörper produzierenden Lymphocyten können entweder durch Fusion mit einer humanen Myelom-Zellinie oder durch Epstein-Barr-Virus (EBV)-Transformation zu Antikörper-produzierenden Hybridom-Zellen immortalisiert werden (Monoclonal Antibody and Immunosensor Technology, A.M. Campbell, Elsevier Verlag 1991; Monoklonale Antikörper, J.H. Peters, H. Baumgarten, Springer Verlag 1990; Monoclonal Antibody Production Techniques and Applications, ed. Lawrence B. Schook, Marcel Dekker Verlag 1987).

Wesentlicher Bestandteil von Rezeptor R3 ist ein Antigen, das mit den zu bestimmenden, gegen ein Antigen aus dem HIV1- bzw. HIV1-SubO- und/oder HIV2-env-Bereich gerichteten Antikörpern spezifisch bindefähig ist. Das Antigen ist bevorzugt bindefähig mit Antikörpern. die gegen gp41 oder gp120 (HIV1) bzw. gp36 oder gp110 (HIV2) gerichtet sind. besonders bevorzugt bindefähig mit Antikörpern, die gegen gp41 bzw. gp36 gerichtet sind. Unter dem Begriff "Antigen" ist ein Molekül zu verstehen, das zu einer spezifischen Bindung mit einem gegen ein HIV-env-Genprodukt gerichteten Antikörper fähig ist. Das Antigen kann dabei dem natürlichem Antigen entsprechen. Es kann also aus dem Virus isoliert oder rekombinant hergestellt worden sein. Es können auch synthetische oder rekombinant hergestellte Peptide eingesetzt werden, die in der Lage sind. die zu bestimmenden Antikörper spezifisch zu binden. Das Antigen kann auch durch andere Liganden wie beispielsweise Lipide oder Zucker derivatisiert worden sein. Auch Aminosäureaustausche (D- und L-Aminosäuren oder verwandte Moleküle) oder Deletionen oder Insertionen von Aminosäuren sind möglich. Weitere, für die Testführung vorteilhafte Modifikationen des Antigens wie z.B. durch die in der WO 96/03652 beschriebene Herstellung von Polyhaptenen, können vom Fachmann leicht herausgefunden werden. Einzige Voraussetzung ist, daß die zu bestimmenden Antikörper trotz der Modifikationen immer noch spezifisch an das modifizierte Antigen binden können. Das heißt. die Bindungsstelle für den oder die Antikörper muß auf dem Antigen, das Bestandteil von R3 ist, erhalten bleiben.

R3 kann entweder direkt an die Festphase gebunden sein, oder die Bindung an die Festphase erfolgt indirekt über ein spezifisches Bindungssystem. Die direkte und die indirekte Bindung von R3 an die Festphase erfolgt analog zu der für den Rezeptor R1 beschriebenen Weise.

Der Rezeptor R4 besteht aus einem Antigen, das mit dem zu bestimmenden, gegen ein Antigen aus dem HIV1- bzw. HIV1-SubO- und/oder HIV2-env-Bereich gerichteten Antikörper spezifisch bindefähig ist und einer Markierung, Das Antigen ist bevorzugt bindefähig mit einem Antikörper, der gegen gp41 oder gp120 bzw. gp36 oder gp110 gerichtet ist, besonders bevorzugt bindefähig mit einem Antikörper. der gegen gp4 1 bzw. gp36 gerichtet ist. Die Bedingungen für das Antigen sowie die Definition des Begriffes sind identisch mit den für den Rezeptor R3 genannten Bedingungen. Bevorzugt werden als "Antigen-Komponenten" der Rezeptoren R3 und R4 die gleichen Antigene eingesetzt. Es ist aber auch möglich, verschiedene Antigene einzusetzen. Es muß jedoch sichergestellt sein, daß beide Antigen-Komponenten aus R3 und R4 mit dem gleichen zu bestimmenden Antikörper bindefähig sind. Voraussetzung ist immer, daß der zu bestimmende Antikörper die beiden Rezeptoren R3 und R4 verbrückt.

Ein weiterer Bestandteil von Rezeptor R4 ist die Markierung. Die Voraussetzungen für die Markierung in R4 sind die gleichen wie die für die Markierung von Rezeptor R2 beschriebenen. Bevorzugt werden für R2 und R4 die gleichen Markierungen verwendet.

Der Rezeptor R5 besteht im wesentlichen aus einem Antigen. das mit dem zu bestimmenden, gegen ein Antigen aus dem pol- oder -gag-Bereich von HIV1, HIV1-SubO und/oder HIV2 gerichteten Antikörper spezifisch bindefähig ist, wobei dies nicht Epitope oder Sequenzen von p24 oder p26 sein dürfen, die mit den Rezeptoren R1 oder R2 reagieren können. Das Antigen ist bevorzugt bindefähig mit einem Antikörper, der gegen Genprodukte des pol-Bereiches gerichtet ist, besonders bevorzugt bindefähig mit einem Antikörper, der gegen die Reverse Transkriptase (RT) gerichtet ist. Besonders geeignet ist hierbei die natürlicherweise vorkommende RT, die als Heterodimer (bestehend aus den beiden Untereinheiten von 51 und 66 kDa) vorkommt und eine möglichst native Struktur besitzt. Die Untereinheiten können aber auch einzeln verwendet werden. Besonders bevorzugt wird eine rekombinant hergestellte aufgereinigte RT verwendet, die von einem Expressionsklon wie er beispielsweise bei Müller et al. in J. Biol. Chem. 264/24: 13975-13978 (1989) beschrieben ist, exprimiert wird. Aufgrund des hohen Homologiegrades auf Aminosäureebene zwischen HIV1- und HIV2-RT von etwa 60% bzw. abschnittweise sogar 100%-iger Übereinstimmung der beiden Proteine reicht die HIV1-RT im allgemeinen aus, um auch von HIV2-Antikörpern erkannt zu werden. Gegebenenfalls kann jedoch auch HIV2-RT allein oder zusätzlich eingesetzt werden. Statt der RT kann auch ein Antigen verwendet werden, das mit einem Antikörper bindefähig ist, der gegen Genprodukte des gag-Bereiches mit Ausnahme der Epitope von p24 oder p26, die mit den Rezeptoren R1 oder R2 reagieren, gerichtet ist. Bevorzugt ist das Antigen bindefähig mit einem Antikörper, der gegen gag-p17 gerichtet ist. Zur Definition des Begriffes "Antigen" gilt das gleiche wie für die Rezeptoren R3 und R4 beschriebene mit Ausnahme der Spezifität: Das Antigen beim Rezeptor R5 ist natürlich ein Molekül, das zu einer spezifischen Bindung mit einem gegen ein HIV-gag- oder pol-Genprodukt gerichteten Antikörper fähig ist.

R5 kann entweder direkt an die Festphase gebunden sein, oder die Bindung an die Festphase erfolgt indirekt über ein spezifisches Bindungssystem. Die direkte und die indirekte Bindung von R5 an die Festphase erfolgt analog zu der für die Rezeptoren R1 und R3 beschriebenen Weise.

Der Rezeptor R6 besteht im wesentlichen aus einem Antigen. das mit dem zu bestimmenden, gegen ein Antigen aus dem pol- oder -gag-Bereich von HIV1. HIV1-SubO und/oder HIV2 gerichteten Antikörper spezifisch bindefähig ist. Das Antigen ist bevorzugt bindefähig mit einem Antikörper, der gegen Genprodukte des pol-Bereiches gerichtet ist, besonders bevorzugt bindefähig mit einem Antikörper, der gegen die Reverse Transkriptase gerichtet ist. Die im Abschnitt zu Rezeptor R5 gemachten Ausführungen bezüglich der bevorzugten Antigen-Eigenschaften gelten auch für R6. Zur Definition des Begriffes "Antigen" gilt das gleiche wie für den Rezeptor R5. Bevorzugt werden als "Antigen-Komponenten" der Rezeptoren R5 und R6 die gleichen Antigene eingesetzt. Es ist aber auch möglich, verschiedene Antigene einzusetzen. Es muß jedoch sichergestellt sein, daß beide Antigen-Komponenten aus R5 und R6 mit dem gleichen zu bestimmenden Antikörper bindefähig sind, Voraussetzung ist immer, daß der zu bestimmende Antikörper die beiden Rezeptoren R5 und R6 verbrückt.

Ein weiterer Bestandteil von Rezeptor R6 ist die Markierung. Die Voraussetzungen für die Markierung in R6 sind die gleichen wie die für die Markierung der Rezeptoren R2 und R4 beschriebenen. Bevorzugt werden für R2, R4 und R6 die gleichen Markierungen verwendet.

Für alle Antigene, die als Rezeptoren R3, R4, R5 oder R6 verwendet werden, gilt, daß sie einzeln oder bevorzugt auch vernetzt als Oligomere oder als Polyhaptene (mit den Antigenen beladene Trägermoleküle oder Trägerpartikel) gemäß der WO 96/03652 eingesetzt werden können. Dadurch wird die IgM-Erkennung verbessert.

Als Proben, die im Nachweisverfahren eingesetzt werden, können alle dem Fachmann bekannten biologischen Flüssigkeiten verwendet werden. Bevorzugt werden als Probe Körpertlüssigkeiten wie Vollblut, Blutserum, Blutplasma, Urin, Speichel etc. eingesetzt.

In den Testansätzen können neben der Probe, der Festphase und den aufgeführten Rezeptoren weitere, je nach Anwendung benötigte Zusätze wie Puffer, Salze. Detergenzien, Proteinzusätze wie beispielsweise RSA und Proteinspaltprodukte wie beispielsweise Pepton vorhanden sein. Die notwendigen Zusätze sind dem Fachmann bekannt oder können von ihm auf einfache Weise herausgefunden werden.

Ein weiterer Gegenstand der Erfindung ist ein Reagenzienkit zum Nachweis einer HIV-Infektion, enthaltend mindestens einen Rezeptor R1, der mit dem p24-Antigen von HIV1, HIV1-SubO und/oder dem p26-Antigen von HIV2 spezifisch bindefähig ist und an eine Festphase gebunden ist oder gebunden werden kann, mindestens einen Rezeptor R2, der mit dem p24-Antigen von HIV1, HIV1-SubO und/oder dem p26-Antigen von HIV2 spezifisch bindefähig ist und eine Markierung trägt, wobei die von R1 und R2 erkannten Epitope verschieden sind, mindestens einen Rezeptor R3, der mit dem zu bestimmenden. gegen ein Antigen aus dem env-Bereich gerichteten HIV1-, HIV1-SubO und/oder HIV2-Antikörper spezifisch bindefähig ist und an eine Festphase gebunden ist oder gebunden werden kann, mindestens einen Rezeptor R4, der mit dem zu bestimmenden, gegen ein Antigen aus dem env-Bereich gerichteten HIV1-, HIV1-SubO und/oder HIV2-Antikörper spezifisch bindefähig ist und eine Markierung trägt, mindestens einen Rezeptor R5, der mit dem zu bestimmenden, gegen ein Antigen aus dem pol- oder gag-Bereich gerichteten HIV1-, HIV1-SubO und/oder HIV2-Antikörper spezifisch bindefähig ist und an eine Festphase gebunden ist oder gebunden werden kann, mindestens einen Rezeptor R6, der mit dem zu bestimmenden, gegen ein Antigen aus dem pol- oder gag-Bereich gerichteten HIV1-, HIV1-SubO oder HIV2-Antikörper spezifisch bindefähig ist und eine Markierung trägt, sowie gegebenfalls weitere übliche Hilfsstoffe.

Weiterhin ist ein Gegenstand der Erfindung ein zuvor beschriebenes Reagenzienkit, das dadurch gekennzeichnet ist. daß als Rezeptoren R1 und R2 Antikörper, die von den Zellinien MAK<p24>M-6A9/5, MAK<p24>M-4B1/1, MAK<p24>M-6D9/4, und/oder MAK<p24>M-2E7/3 produziert werden, eingesetzt werden.

Ebenfalls offenbart ist ein Reagenzienkit zum Nachweis von Antikörpern gegen HIV, in dem die Rezeptoren R3, R4, R5 und R6, sowie gegebenfalls weitere übliche Hilfsstoffe enthalten sind. Die Rezeptoren R1 und R2 sind nicht enthalten.

Auch ein Gegenstand der Erfindung ist ein Teststreifen zum Nachweis einer HIV-Infektion. auf dem mindestens ein Rezeptor R1, der mit dem p24-Antigen von HIV1. HIV1-SubO und/oder dem p26-Antigen von HIV2 spezifisch bindefähig ist und an eine Festphase gebunden ist oder gebunden werden kann, mindestens einen Rezeptor R2, der mit dem p24-Antigen von HIV1, HIV1-SubO und/oder dem p26-Antigen von HIV2 spezifisch bindefähig ist und eine Markierung trägt, wobei die von R1 und R2 erkannten Epitope verschieden sind, mindestens ein Rezeptor R3, der mit dem zu bestimmenden, gegen ein Antigen aus dem env-Bereich gerichteten HIV1-, HIV1-SubO und/oder HIV2-Antikörper spezifisch bindefähig ist und an eine Festphase gebunden ist oder gebunden werden kann, mindestens ein Rezeptor R4, der mit dem zu bestimmenden, gegen ein Antigen aus dem env-Bereich gerichteten HIV1-, HIV1-SubO und/oder HIV2-Antikörper spezifisch bindefähig ist und eine Markierung trägt, mindestens ein Rezeptor R5, der mit dem zu bestimmenden, gegen ein Antigen aus dem pol- oder gag-Bereich gerichteten HIV1-, HIV1-SubO und/oder HIV2-Antikörper spezifisch bindefähig ist und an eine Festphase gebunden ist oder gebunden werden kann, mindestens ein Rezeptor R6, der mit dem zu bestimmenden, gegen ein Antigen aus dem pol- oder gag-Bereich gerichteten HIV1-, oder HIV2-Antikörper spezifisch bindefähig ist und eine Markierung trägt, sowie gegebenfalls weitere übliche Hilfsstoffe aufgebracht sind.

Weiterhin offenbart ist ein Teststreifen zum Nachweis von Antikörpern gegen HIV, in dem die Rezeptoren R3, R4. R5 und R6. sowie gegebenfalls weitere übliche Hilfsstoffe enthalten sind. Die Rezeptoren R1 und R2 sind nicht enthalten.

In den Figuren 1 bis 5 ist folgendes dargestellt:
Fig. 1: Plateaubestimmung des monoklonalen Antikörpers MAK<p24>M-6A9/5: Die Bestimmung der optimalen MAK-Einsatzkonzentration für die Epitopkartierung wird gemäß dem in Beispiel 4.2 beschriebenen Verfahren durchgeführt.
Fig. 2: Plateaubestimmung des monoklonalen Antikörpers MAK<p24>M-4B1/1
Fig. 3: Plateaubestimmung des monoklonalen Antikörpers MAK<p24>M-6D9/4
Fig. 4: Plateaubestimmung des monoklonalen Antikörpers MAK<p24>M-2E7/3
Fig. 5: p24-Epitopkartierung; Reaktion der <p24>-MAKs mit verschiedenen Peptidmischungen gemäß Beispiel 4.2.3

Die folgenden Beispiele erläutern die Erfindung weiter.

### Beispiele

### Beispiel 1: Durchführung des Kombitests

Die Versuchsdurchführung erfolgt analog zu der in der Packungsbeilage des Enzymun-Test® Anti-HIV 1+2+SubtypO (Best.Nr. 1557319. Boehringer Mannheim GmbH. Deutschland) beschriebenen Vorgehensweise. Bis auf d Rezeptoren R1 bis R6 werden die Reagenzien und Puffer des Enzymun-Test® Anti-HIV 1+2+SubtypO der Boehringer Mannheim GmbH eingesetzt. Der Test wird bei 25°C auf dem Gerät ES700 (Hersteller: Boehringer Mannheim GmbH, Deutschland) in einem Probenvolumen von 100 µl in streptavidinbeschichteten Teströhrchen nach dem Prinzip des 2-Schritt-Sandwich-ELISA durchgeführt. Im einzelnen werden folgende Reagenzien verwendet:
- Inkubationspuffer: Tris 25 mM pH 7,5; Rinderserumbestandteile
- Konjugatpuffer: Tris 25 mM pH 7,5; Rinderserumbestandteile
- Konjugat: Peroxidase(POD)-markierte Anti-Digoxigenin-Antikörper vom Schaf
- Substrat: ABTS®-Substratlösung (2,2'-Azino-di[3-ethylbenzthiazolin-sulfonat] 1,9 mmol/l in 100 mmol/l Phosphat/Citrat-Puffer, pH 4.4, Natriumperborat 3,2 mmol/l

Als Rezeptoren R1 bis R6 werden eingesetzt (Bi ist Abkürzung für Biotin. Dig ist Abkürzung für Digoxigenin):
- R1: MAK<p24>M-6A9/5-IgG-Bi, MAK<p24>M-4B1/1-IgG-Bi
- R2: MAK<p24>M-6D9/4-IgG-Dig, MAK<p24>M-2E7/3-IgG-Dig
- R3: gp36-, gp41-Peptide-Bi und Polyhaptene-Bi entsprechend den im Enzymun®-Test Anti-HIV1+2+SubO, Best.Nr. 1557319, Boehringer Mannheim Deutschland verwendeten Peptiden
- R4: gp36-, gp41-Peptide-Dig und Polyhaptene-Dig entsprechend den im Enzymun®-Test Anti-HIV1+2+SubO, Best.Nr. 1557319, Boehringer Mannheim Deutschland verwendeten Peptiden
- R5: RT-Bi hergestellt aus RT, Best.Nr. 1465333, Boehringer Mannheim Deutschland
- R6: RT-Dig hergestellt aus RT, Best.Nr. 1465333, Boehringer Mannheim Deutschland Die hierzu notwendigen Derivatisierungen mit aktivierten Biotin- und Digoxigenin-Derivaten wurden nach dem Fachmann aus Lehrbüchern bekannten Verfahren durchgeführt. Für die Derivatisierung der RT oder der Antikörper eignen sich bevorzugt D-Biotin-ε-aminohexanoyl-N-hydroxy-succinimid und Digoxigenin-3-carboxymethylether-ε-aminohexanoyl-N-hydroxy-succinimid.

Die Rezeptoren R1 bis R6 (Antikörper, Polyhaptene und RT jeweils 50-300 ng/ml, Peptide 5-50 ng/ml) werden zusammen mit dem Inkubationspuffer in die streptavidinbeschichteten Röhrchen gegeben und 120 min inkubiert. Nach mehreren Waschvorgängen und einer 60-minütigen Inkubation mit dem Konjugat wird die Substratlösung zugegeben und die Extinktionswerte der entstandenen Farblösungen nach 60 min bei 422 nm photometrisch bestimmt.

### Beispiel 2: Vergleich verschiedener Kombitests

Um die Erfindung mit dem Stand der Technik zu vergleichen, wurden kommerzielle HIV-Seroconversionspanel der Firma Boston Biomedica Inc., Bridgewater, USA (BBI) vermessen und mit den von BBI gelieferten Daten verglichen (Tabelle 1).
- BBI Panel N:: Best.Nr. PRB914
- BBI Panel W:: Best.Nr. PRB923
- BBI Panel AG:: Best.Nr. PRB932
- HIV-Ag Test:: Abbott Monodonal HIV Ag, Prod.Nr. 2A81 (Daten von BBI)
- Anti-HIV Test 1:: Abbott HIV1/2, Prod.Nr. 3A77 (Daten von BBI)
- Anti-HIV Test 2:: Gen. Sys. HIV1/2, Prod.Nr. 0230 (Daten von BBI)
- Kombitest 1:: Anti-p24-Antikörper, gp41 und gp36 (Beschreibung siehe unten)
- Kombitest 2:: Anti-p24-Antikörper und RT (Beschreibung siehe unten)
- Kombitest 3:: Anti-p24-Antikörper, gp41, gp36 und RT (Beschreibung siehe unten)

Die drei Kombiteste wurden unter identischen Bedingungen mit den gleichen Reagenzien mit Ausnahme der unterschiedlichen Antigene durchgeführt. Die Testdurchführung erfolgte analog zu Beispiel 1. Als Basis dienten (wie in Beispiel 1) die Reagenzien und die 25°C Applikation des käuflichen Produktes Enzymun-Test® Anti-HIV1+2+SubtypO (Best.Nr. 1557319, Boehringer Mannheim, Deutschland), Für die Kombitests 1 bis 3 wurden folgende Antikörper bzw. Antigene verwendet:
- Kombitest 1:
   MAK<p24>M-6A9/5-IgG-Bi. MAK<p24>M-4B1/1-IgG-Bi und biotinylierte synthetische Peptide und Polyhaptene (gp41 und gp36) aus dem Enzymun-Test® Anti-HIV1+2+SubtypO. MAK<p24>M-6D9/4-IgG-Dig, MAK<p24>M-2E7/3-IgG-Dig und digoxigenylierte synthetische Peptide und Polyhaptene (gp41 und gp36) aus dem Enzymun-Test® Anti-HIV 1+2+SubtypO
- Kombitest 2:
   MAK<p24>M-6A9/5-IgG-Bi. MAK<p24>M-4B1/1-IgG-Bi und biotinylierte rekombinante HIV-Reverse Transkriptase
   MAK<p24>M-6D9/4-IgG-Dig, MAK<p24>M-2E7/3-IgG-Dig und digoxigenylierte rekombinante HIV-Reverse Transkriptase
- Kombitest 3 (erfindungsgemäß):
   R1: MAK<p24>M-6A9/5-IgG-Bi, MAK<p24>M-4B1/1-IgG-Bi,
   R3: biotinylierte synthetische Peptide und Polyhaptene (gp41 und gp36) aus dem Enzymun-Test® Anti-HIV 1+2+SubtypO
   R5: biotinylierte rekombinante HIV-Reverse Transkriptase
   R2: MAK<p24>M-6D9/4-IgG-Dig. MAK<p24>M-2E7/3-IgG-Dig.
   R4: digoxigeninylierte synthetische Peptide und Polyhaptene(gp41 und gp36) aus dem Enzymun-Test® Anti-HIV 1+2+SubtypO
   R6: digoxigenylierte rekombinante HIV-Reverse Transkriptase

Für alle Tests ist das Verhältnis von Signal zu Cut-off-Wert angegeben (Cut-off Index. coi). Die Daten sind folgendermaßen zu interpretieren: Werte kleiner 0.9 sind negativ, Werte zwischen 0,9 und 1,0 sind im Graubereich und Werte größer gleich 1,0 sind positiv. In der Spalte "Tag" ist die Anzahl der Tage eingetragen, die seit der ersten aufgeführten Blutabnahme vergangen sind. Die eingesetzten Panel enthalten unterschiedliche Analyten (Antigene und Antikörper), die in der Serokonversionsphase nach den unter "Tag" angegebenen Zeiten auftreten.

**Tabelle 1:**

| **Vergleich verschiedener HIV-Nachweistests** | | | | | | | |
|---|---|---|---|---|---|---|---|
| **Panel** | **Tag** | **HIV-Ag Test** | **Anti-HIV Test 1** | **Anti-HIV Test 2** | **Kombitest 1** | **Kombitest 2** | **Kombitest 3** |
| **BBI Panel N** | **0** | 0,4 | **8,4** | 0,8 | **8,9** | 0,4 | **11,3** |
| | **4** | 0,5 | **7,8** | **1,1** | **5.7** | 0,1 | **7,4** |
| | **7** | 0,5 | **8,0** | **1,4** | **3,5** | 0,1 | **4.5** |
| | **25** | 0,4 | **12,8** | **2,8** | **1,4** | 0,8 | **3,3** |
| | **31** | 0,4 | **11,4** | **3,0** | **1,1** | **2,3** | **4,3** |
| **BBI Panel W** | **0** | 0.4 | 0,1 | 0,2 | 0,3 | 0,2 | 0,3 |
| | **7** | 0,4 | 0,1 | 0,2 | 0,3 | 0,1 | 0,3 |
| | **12** | 0,5 | 0,1 | 0,2 | 0.3 | 0,1 | 0,3 |
| | **14** | 0,4 | 0,1 | 0,2 | 0,3 | 0,1 | 0.3 |
| | **28** | 0,4 | 0,1 | 0,2 | 0,3 | 0,1 | 0,3 |
| | **30** | 0,4 | 0,1 | 0,2 | 0,3 | 0,1 | 0,3 |
| | **35** | 0,4 | 0,1 | 0,2 | 0,3 | 0,1 | 0,3 |
| | **37** | **1,0** | 0,1 | 0,2 | 0,4 | 0,2 | 0,4 |
| | **47** | **23,3** | **1,4** | **0,2** | **8,4** | **10,9** | **14,3** |
| | **84** | 0,4 | **7,8** | **5,5** | **1,2** | **24,3** | **16,6** |
| | **86** | 0,4 | **7,6** | **5,5** | **1,2** | **24,3** | **16,1** |
| | **145** | 0,4 | **17,2** | **4,0** | **6,5** | **24,3** | **24,8** |
| | **161** | 0,5 | **17,2** | **3,1** | **6,4** | **24,3** | **24,8** |
| **BBI Panel AG** | **0** | 0,4 | 0,1 | 0,1 | 0,3 | 0,2 | 0,3 |
| | **3** | 0,4 | 0,1 | 0,1 | 0,3 | 0,5 | 0,3 |
| | **13** | 0,4 | 0,2 | 0,2 | 0,3 | 0,2 | 0,3 |
| | **27** | **4,6** | **1,1** | **0,2** | **1,1** | **1,0** | **1,1** |
| | **34** | **6,1** | **12,5** | **2,6** | **10,0** | **9,2** | **21,9** |
| | **50** | **1,9** | **1,7** | **3,4** | **4,7** | **24,2** | **14,4** |
| | **78** | **3,2** | **0,6** | **1,3** | **2,3** | **24,3** | **12,7** |
| | **163** | **1,8** | **2,1** | 0,9 | **0,8** | **17,3** | **7.6** |
| | **194** | **1,0** | **8,6** | 0,8 | **0,8** | **13,1** | **6,4** |

Es zeigt sich, daß der erfindungsgemäße Kombitest (Kombitest 3) im Unterschied zu den Vereleichstests alle Phasen der Infektion lückenlos delektiert.

### Beispiel 3: Erkennung von HIV1-Virusisolaten verschiedener Subtypen

Durch die Verwendung der vier ertindungsgemäßen Antikörper ist eine breite HIV1-Subtyperkennune gewährleistet. Die Testführung, Reagenzzusammensetzung und Auswertung ist identisch zu dem erfindungsgemäßen Kombitest 3 aus Beispiel 2. Als Proben wurden alle unten aufgeführten HIV1-Virusisolate (Gruppe M und Subtyp O) 1:100 in Humanserum verdünnt, da die unverdünnten Isolate alle das gleich große Maximalsignal ergaben und somit keine Unterschiede zu sehen waren.

**Tabelle 2:**

| HIV1-Virus | Subtyp | cut-off Index |
|---|---|---|
| MP20 | Subtyp A | 9,0 |
| MP33 | Subtyp A | 22,2 |
| MP95 | Subtyp A | 19,2 |
| MP97 | Subtyp A | 9,1 |
| MP157 | Subtyp A | 36,0 |
| MP8 | Subtyp B | 23,4 |
| MP13 | Subtyp B | 18,7 |
| MP22 | Subtyp B | 22,5 |
| MP51 | Subtyp B | 20,9 |
| MP77 | Subtyp B | 30,5 |
| MP37 | Subtyp C | 9,1 |
| MP40 | Subtyp C | 22,2 |
| MP41 | Subtyp C | 9,1 |
| MP76 | Subtyp C | 18,2 |
| MP148 | Subtyp C | 16,4 |
| VI693 | Subtyp D | 16,9 |
| VI722 | Subtyp D | 10,5 |
| VI824 | Subtyp D | 16,3 |
| VI979 | Subtyp D | 23,2 |
| VI1091 | Subtyp D | 14,9 |
| VI1249 | Subtyp E | 23,4 |
| MP38 | Subtyp E | 19,4 |
| MP48 | Subtyp E | 21,0 |
| MP121 | Subtyp E | 22,6 |
| MP126 | Subtyp E | 2,5 |
| V1850 | Subtyp F | 11,0 |
| VI961 | Subtyp F | 22,3 |
| VI1126 | Subtyp F | 11,4 |
| VI1267 | Subtyp F | 15,3 |
| VI310 | Subtyp F | 21,4 |
| MP84 | Subtyp F | 12,2 |
| VI1197 | Subtyp G | 29,9 |
| VI991 | Subtyp H | 8,1 |
| VI997 | Subtyp H | 10,0 |
| MP331 | Subtyp O | 4,2 |

Es zeigt sich, daß der erfindungsgemäße Kombitest die Antigene und Antikörper aller HIV 1-Subtypen sicher detektiert.

### Beispiel 4: Epitopkartierung der monoklonalen Antikörper gegen p24

### 4.1. Peptidsynthese

Die entsprechenden Teilsequenzen der Aminosäuresequenz des HIV-p24-Virusproteins werden mittels Fluorenylmethyloxycarbonyl-(Fmoc)-Festphasenpeptidsynthese an einem Batch-Peptidsynthesizer, z.B. von Applied Biosystems A431 oder A433, hergestellt. Dazu werden jeweils 4.0 Äquivalente von folgenden Fmoc-Aminosäurederivaten verwendet:

**Tabelle 3:**

| | |
|---|---|
| A | Fmoc-Ala-OH |
| C | Fmoc-Cys(Trt)-OH |
| D | Fmoc-Asp(tBu)-OH |
| E | Fmoc-Glu(tBu)-OH |
| F | Fmoc-Phe-OH |
| G | Fmoc-Gly-OH |
| H | Fmoc-His(Trt)-OH |
| I | Fmoc-Ile-OH |
| K | Fmoc-Lys(Phenylacetyl)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBu)-OH |
| U | Fmoc-ßAlanin |
| V | Fmoc-Val-OH |
| W | Fmoc-Trp-OH |
| X | Boc-Lys(Fmoc)-OH |
| Y | Fmoc-Tyr(tBu)-OH |
| Z | Fmoc-Aminocapronsäure |

Die Aminosäuren oder Aminosäurederivate werden in N-Methylpyrolidon gelöst. Das Peptid wird an 400-500 mg 4-(2',4'-Dimethoxyphenyl-Fmoc-Aminomethyl)-Phenoxy-Harz (Tetrahedron Letters 28 (1987), 2107) mit einer Beladung von 0.4 - 0.7 mmol/g aufgebaut (JACS 95 (1973), 1328). Die Kupplungsreaktionen werden bezüglich Fmoc-Aminosäurederivat mit 4 Äquivalenten Dicyclohexylcarbodiimid und 4 Äquivaltenen N-Hydroxybenzotriazol in Dimethylformamid als Reaktionsmedium während 20 min durchgeführt. Nach jedem Syntheseschritt wird die Fmoc-Gruppe mit 20 %igem Piperidin in Dimethylformamid in 20 min abgespalten. Enthalten die Peptide eine intramolekulare Disulfidbrücke. so wird die Fmoc-geschützte, Peptidsequenz vor der Kupplung des artifiziellen Spacers mit Jod in Hexafluorisopropanol/Dichlormethan (Kober et al.. The Peptide Academic Press. New York. 1981. pp 145-47) an der Festphase oxidiert und anschließend die N-terminale Fmoc-Schutzeruppe abgespalten, und der Spacer sowie das N-terminale Biotin gekuppelt.

Die Freisetzung des Peptides vom Syntheseharz und die Abspaltung der säurelabilen Schutzgruppen - mit Ausnahme der Phenylacetylschutzgruppe am Lysin - erfolgt mit 20 ml Trifluoressigsäure. 0.5 ml Ethandithiol, 1 ml Thioanisol. 1.5 g Phenol und 1 ml Wasser in 40 min bei Raumtemperatur. Die Reaktionslösung wird anschließend mit 300 ml gekühltem Diisopropylether versetzt und zur vollständigen Fällung des Peptides 40 min bei 0°C gehalten. Der Niederschlag wird abfiltriert, mit Diisopropylether nachgewaschen, mit wenig 50 %iger Essigsäure gelöst und lyophilisiert. Das erhaltene Rohmaterial wird mittels präparativer HPLC an Delta-PAK RP C18-Material (Säule 50 x 300 mm, 100 Å. 15 µ) über einen entsprechenden Gradienten (Eluent A: Wasser, 0.1 % Trifluoressigsäure. Eluent B: Acetonitril, 0.1 % Trifluoressigsäure) in ca 120 min aufgereinigt. Die Identität des eluierten Materials wird mittels Ionenspray-Massenspektrometrie geprüft.

**Tabelle 4: Peptid-Sequenzen der p24-Epitope**

| **Peptid** | **Sequenz** | **SEQ ID NO** |
|---|---|---|
| 1 | DKGNSSQVSQNYPIVQNLQGQMVHQ | 1 |
| 2 | NYPIVQNLQGQMVHQAISPRTLNAW | 2 |
| **3** | **QMVHQAISPRTLNAWVKVIEEKAFS** | **3** |
| 4 | TLNAWVKVIEEKAFSPEVIPMFSAL | 4 |
| 5 | EKAFSPEVIPMFSALSEGATPQDLN | 5 |
| 6 | MFSALSEGATPQDLNTMLNTVGGHQ | 6 |
| 7 | PQDLNTMLNTVGGHQAAMQMLKETI | 7 |
| 8 | VGGHQAAMQMLKETINEEAAEWDRV | 8 |
| **9** | **LKETINEEAAEWDRVHPVHAGPIAP** | **9** |
| 10 | EWDRVHPVHAGPIAPGQMREPRGSD | 10 |
| 11 | GPIAPGQMREPRGSDIAGTTSTLQE | 11 |
| 12 | PRGSDIAGTTSTLQEQIGWMTNNPP | 12 |
| 13 | STLQEQIGWMTNNPPIPVGEIYKRW | 13 |
| 14 | TNNPPIPVGEIYKRWIILGLNKIVR | 14 |
| 15 | IYKRWIILGLNKIVRMYSPVSILDI | 15 |
| 16 | NKIVRMYSPVSILDIRQGPKEPFRD | 16 |
| **17** | **SILDIRQGPKEPFRDYVDRFYKTLR** | **17** |
| 18 | EPFRDYVDRFYKTLRAEQASQEVKN | 18 |
| 19 | YKTLRAEQASQEVKNWMTETLLVQN | 19 |
| 20 | QEVKNWMTETLLVQNANPDCKTILK | 20 |
| 21 | LLVQNANPDCKTILKALGPAATLEE | 21 |
| 22 | KTILKALGPAATLEEMMTACQGVGG | 22 |
| 23 | ATLEEMMTACQGVGGPGHKARVLAE | 23 |
| 24 | QGVGGPGHKARVLAEAMSQVTNSAT | 24 |
| 25 | RVLAEAMSQVTNSATIMMQRGNFRN | 25 |
| 26 | TNSATIMMQRGNFRNQKKTKCFNC | 26 |
| 27 | GNFRNQKKTVKCFNCGKEGHIAKNC | 27 |
| 28 | KCFNCGKEGHIAKNCRAPRLKGCWK | 28 |
| 29 | IAKNCRAPRLKGCWKCGKEGHQMKD | 29 |
| 30 | KGCWKCGKEGHQMKDCTERQANFLGKI | 30 |
| | | |
| **3A** | **QAISPRTLNAWVKVI** | **31** |
| 3B | ISPRTLNAWVK | 32 |
| 9A | INEEAAEWDRVHPVH | 33 |
| **9B** | **EEAAEWDRVHP** | **34** |
| 17A | IRQGPKEPFRDYVDR | 35 |
| 17B | QGPKEPFRDYV | 36 |

### 4.2. Epitopkartierung

### 4.2.1. Immunologischer Test allgemein

Die Epitopcharakterisierung wurde analog dem Boehringer Mannheim Enzymun®-Test Anti-HIV 1+2 (BM 1 165 062) durchgeführt. Das Testprinzip ist ein 2-Schritt-Sandwich-ELISA mit Streptavidin-Technologie. Jedoch wurden statt der Humanseren die verschiedenen monoklonalen <p24>Antikörper eingesetzt und mit den jeweils biotinylierten Peptiden oder rekombinantem.p24 vermessen. Die Detektion erfolgt über <Maus IgG>-POD (BM 1 431 323). Alle anderen Zusatzreagentien, wie Waschlösung, Streptavidintubes, ABTS usw. wurden beibehalten. Die Messungen wurden an den Enzymun®-Analysen-Automaten ES 22 oder ES 600 von Boehringer Mannheim durchgeführt.

### 4.2.2. Plateaubestimmung

Die Bestimmung der optimalen MAK-Einsatzkonzentration für die Epitopkartierung wurde im oben beschriebenen Testkonzept durchgeführt. Als Antigen wurde biotinyliertes rekombinantes p24 (Einsatzmenge 200 ng/ml) verwendet.

**Tabelle 5:**

| | MAK-Konzentration | Signal |
|---|---|---|
| | c [µg/ml] | mE |
| MAK<p24>-6A9/5 | 0 | 3 |
| | 1 | 208 |
| | 5 | 856 |
| | 10 | 1231 |
| | 20 | 1665 |
| | 25 | 1718 |
| MAK<p24>-6D9/4 | 0 | 2 |
| | 1 | 386 |
| | 5 | 1239 |
| | 10 | 1835 |
| | 20 | 2425 |
| | 25 | 2531 |
| MAK<p24>-2E7/3 | 0 | 5 |
| | 1 | 322 |
| | 5 | 1148 |
| | 10 | 1708 |
| | 20 | 2292 |
| | 25 | 2461 |
| MAK<p24>-4B1/1 | 0 | 9 |
| | 10 | 1205 |
| | 25 | 1944 |
| | 50 | 2116 |
| | 75 | 2451 |
| | 100 | 2537 |

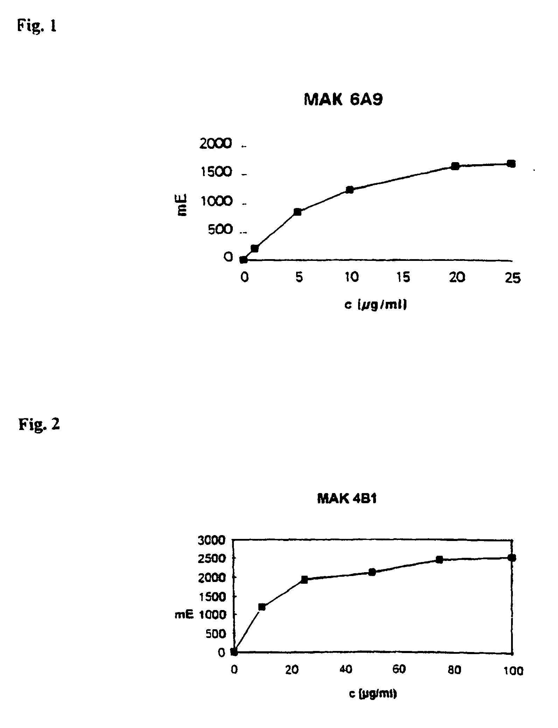

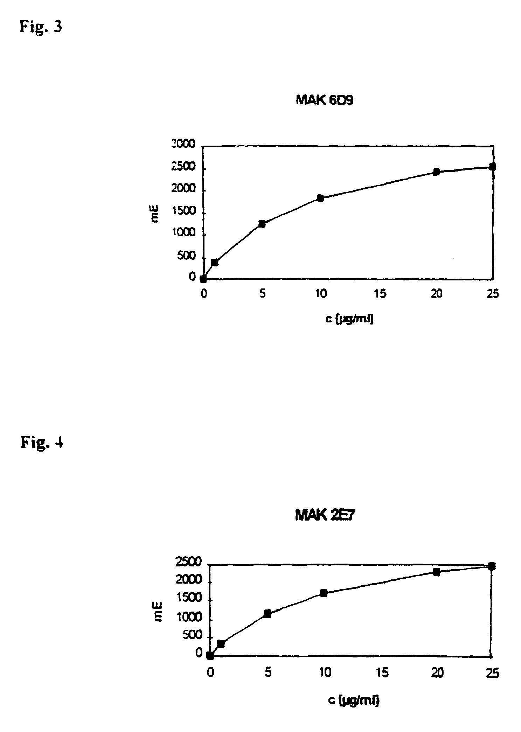

Die Plateaubestimmung ist in den Fig. 1 bis 4 graphisch dargestellt.

### 4.2.3. Epitopkartierung

Die Kartierung wurde mit 25 Aminosäuren langen, N-terminal biotinylierten Peptiden, die im 10er Raster über die HIV1 Consensus B-Sequenz von p24 synthetisiert wurden (Peptide SEQ ID NO: 1-30), durchgeführt. Zuerst wurde eine Grobkartierung mit 6 verschiedenen Peptidmischungen A-F (Einsatzmenge pro Peptid 60 ng/ml), die jeweils 5 verschiedene biotinylierte Peptide enthalten, im indirekten Testkonzept vermessen.

| | | | | | |
|---|---|---|---|---|---|
| **Mischung A:** | 1 | **Mischung B:** | 6 | **Mischung C:** | 11 |
| | 2 | | 7 | | 12 |
| | 3 | | 8 | | 13 |
| | 4 | | 9 | | 14 |
| | 5 | | 10 | | 15 |
| | | | | | |
| **Mischung D:** | 16 | **Mischung E:** | 21 | **Mischung F:** | 26 |
| | 17 | | 22 | | 27 |
| | 18 | | 23 | | 28 |
| | 19 | | 24 | | 29 |
| | 20 | | 25 | | 30 |

**Tabelle 6: Reaktivität der Antikörper mit Peptidmischung A -F**

| | **MAK 6A9/5** | | **MAK 4B1/1** | | **MAK 6D9/4** | | **MAK 2E7/3** | |
|---|---|---|---|---|---|---|---|---|
| | [mE] | % | [mE] | % | [mE] | % | [mE] | % |
| Standardantigen | 2098 | 100 | 2402 | 100 | 2966 | 100 | 2903 | 100 |
| Mischung A | 6 | 0 | 29 | 1 | 925 | **31** | 5 | 0 |
| Mischung B | 8 | 0 | 22 | 1 | 100 | 3 | 2696 | **93** |
| Mischung C | 15 | 1 | 31 | 1 | 22 | 1 | 5 | 0 |
| Mischung D | 22 | 1 | 1152 | **48** | 41 | 1 | 4 | 0 |
| Mischung E | 15 | 1 | 17 | 1 | 16 | 1 | 2 | 0 |
| Mischung F | 24 | 1 | 18 | 1 | 17 | 1 | 3 | 0 |

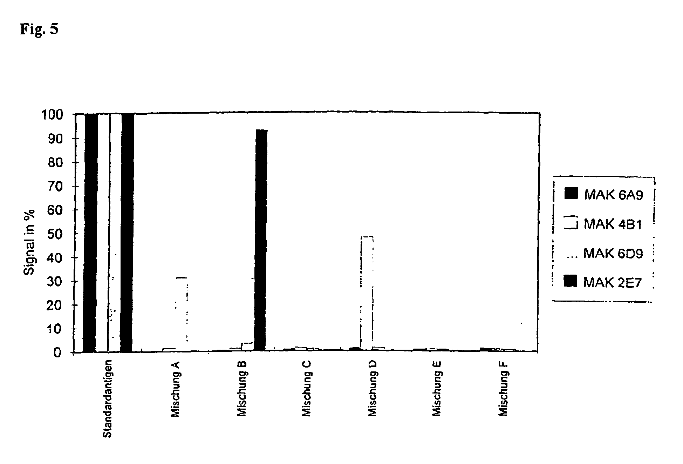

Ergebnis der Grobkartierung: 3 MAKs reagieren spezifisch mit jeweils einer Peptidmischung (graphische Darstellung siehe Fig. 5).

### 4.2.4. Feinkartierung

In der Feinkartierung wurden die Peptide (Einsatzmenge 100 ng/ml) aus den reaktiven Mischungen einzeln im indirekten Testkonzept eingesetzt:

**Tabelle 7:**

| **MAK<p24>M-4B1/1 mit Einzelantigenen aus Mischung D** | | | | | | | | | | | | |
|---|---|---|---|---|---|---|---|---|---|---|---|---|
| | rec p24 | | Peptid 16 | | **Peptid 17** | | Peptid 18 | | Peptid 19 | | Peptid 20 | |
| | mE | | mE | | mE | | mE | | mE | | mE | |
| MAK 4B1 | 2089 | 100% | 3 | 0% | 297 | **14%** | 2 | 0% | 3 | 0% | 0 | 0% |

**Tabelle 8:**

| **MAK<p24>M-2E7/3 mit Einzelantigenen aus Mischung B** | | | | | | | | | | | | |
|---|---|---|---|---|---|---|---|---|---|---|---|---|
| | rec p24 | | Peptid 6 | | Peptid 7 | | Peptid 8 | | **Peptid 9** | | Peptid 10 | |
| | mE | | mE | | mE | | mE | | mE | | mE | |
| MAK 2E7 | 2111 | 100% | 6 | 0% | 3 | 0% | 23 | 1% | 2103 | **99.6%** | 5 | 0% |

**Tabelle 9:**

| **Tabelle 9: MAK<p24>M-6D9/4 mit Einzelantigenen aus Mischung A** | | | | | | | | | | | | |
|---|---|---|---|---|---|---|---|---|---|---|---|---|
| | rec p24 | | Peptid 1 | | Peptid 2 | | **Peptid 3** | | Peptid 4 | | Peptid 5 | |
| | mE | | mE | | mE | | mE | | mE | | mE | |
| MAK 6D9 | 2282 | 100% | 3 | 0% | 23 | 1% | 922 | **40%** | 5 | 0% | 4 | 0% |

**Tabelle 10: reaktive Peptid-Sequenzen:**

| **Peptid SEQ ID NO** | **Sequenz** | **Ergebnis** |
|---|---|---|
| **3** | **QMVHQAISPRTLNAWVKVIEEKAFS** | **reaktiv mit MAK<p24>M-6D9** |
| **9** | **LKETINEEAAEWDRVHPVHAGPIAP** | **reaktiv mit MAK<p24>M-2E7/3** |
| **17** | **SILDIRQGPKEPFRDYVDRFYKTLR** | **reaktiv mit MAK<p24>M-4B1/1** |

**Tabelle 11: Sequenzvergleich der Epitope von p24 HIV I Consensus B und Subtyp O**

| | | |
|---|---|---|
| **MAK 6D9** | QMVHQAISPRTLNAMVKVIEEKAFS | Consensus B |
| | QMVHQAISPRTLNAWVKAIEEKAFN | Subtyp O |
| | | |
| **MAK 2E7** | LKETINEEAAEWDRVHPVHAGPIAP | Consensus B |
| | LKEVINEEA?EWDRTHPP??GPLPP | Subtyp O |
| | | |
| **MAK 4B1** | SILDIRQGPKEPFRDYVDRFYKTLR | Consensus B |
| | SILDI?QGPKEPFRDYVDRFYKTLR | Subtyp O |

Laut Sequenzvergleich sollten die MAKs 6D9 und 4B 1 die Kreuzreaktion zu p24 (SubO) ermöglichen, da hier die größte Homologie in der Sequenz ist.

Zur weiteren Einschränkung des Epitops wurde nochmals eine Feinkartierung mit den Peptiden 3 (= SEQ ID NO 3), 3A (= SEQ ID NO 31), 3B (= SEQ ID NO 32), und MAK<p24>M-6D9/4; Peptide 9 (= SEQ ID NO 9), 9A (= SEQ ID NO 33), 9B (= SEQ ID NO 34) und MAK<p24>M-2E7/3; sowie Peptide 17 (= SEQ ID NO 17), 17A (= SEQ ID NO 35), 17 B (= SEQ ID NO 36) und MAK<p24>M-4B1/1 durchgeführt.

**Tabelle 12:**

| **MAK<p24>M-4B1/1 mit Einzelantigenen 17, 17A und 17B** | | | |
|---|---|---|---|
| SEQ ID NO: | **Peptid 17 17** | Peptid 17A 35 | Peptid 17B 36 |
| | mE | mE | mE |
| MAK4B1/1 | **612** | 10 | 7 |

**Tabelle 13:**

| **MAK<p24>M-2E7/3 mit Einzelantigenen 9, 9A und 9B** | | | |
|---|---|---|---|
| SEQ ID NO: | Peptid 9 9 | Peptid 9A 33 | **Peptid 9B 34** |
| | mE | mE | mE |
| MAK 2E7/3 | 1439 | 1330 | **1399** |

**Tabelle 14:**

| **MAK<p24>M-6D9/4 mit Einzelantigenen 3, 3A, 3B** | | | |
|---|---|---|---|
| SEQ ID NO: | Peptid 3 3 | **Peptid 3A 31** | Peptid 3B 32 |
| | mE | mE | mE |
| MAK 6D9/4 | 958 | **866** | 448 |

Daraus folgt, daß das Epitop des Antikörpers MAK<p24>M-4B1/1 nicht weiter eingeschränkt werden kann. Das Epitop von MAK<p24>M-2E7/3 kann auf die Sequenz

| | |
|---|---|
| 9B (SEQ ID NO: 34) | EEAAEWDRVHP |

minimiert werden. Das Epitop von MAK<p24>M-6D9/4 kann auf die Sequenz

| | |
|---|---|
| 3A (SEQ ID NO: 31) | QAISPRTLNAWVKVI |

ohne größere Signalverluste verkürzt werden kann.

### Beispiel 5: HIV-Subtypenerkennung durch die monoklonalen Anti-p24-Antikörper

Der Test wurde entsprechend Beispiel 1 und 2 nach den Vorschriften des Enzymun-Test® Anti-HIV1-2+SubtypO (Best.Nr. 1557319, Boehringer Mannheim, Deutschland) durchgeführt. Zur Austestung der HIV-Subtypenerkennung wurden jedoch nur die Rezeptoren R1 und R2, d.h. die monoklonalen Antikörper gegen p24 verwendet. Es wurden jeweils 250 ne MAK/ml eingesetzt.

**Tabelle 15:**

| **Erkennung von p24 (HIV1 und HIV1-SubO) durch die MAKs** | | | | | |
|---|---|---|---|---|---|
| Rezeptor R1 | MAK 4B1/1 | MAK 2E7/3 | MAK 4B1/1 | MAK 6A9 | MAK 4B1/1 MAK 6A9/5 |
| Rezeptor R2 | MAK 2E7/3 | MAK 6D9/4 | MAK 6D9/4 | MAK 6D9/4 | MAK 2E7/3 MAK 6D9/4 |
| Probe | | | | | |
| SN 1806 (HIV1 Subtyp O) | 0,1 (negativ) | 0,1 (negativ) | 1,2 (positiv) | 0,1 (negativ) | 1,2 (positiv) |
| SN 1807 (HIV1 Subtyp O) | 0,1 (negativ) | 0,1 (negativ) | 1,7 (positiv) | 0,1 (negativ) | 1,7 (positiv) |
| SN 1796 (HIV1) | 1,1 (positiv) | 0,1 (negativ) | 0,1 (negativ) | 0,6 (negativ) | 1,2 (positiv) |
| SN 1799 (HIV1) | 0,9 (grenzwertig) | 1,3 (positiv) | 1,2 (positiv) | 1,1 (positiv) | 1,4 (positv) |
| SN 1801 (HIV1) | 0,8 (grenzwertig) | 1,7 (positiv) | 0,8 (grenzwertig) | 1,3 (positiv) | 1,7 (positiv) |
| SN 1803 (HIV1) | 1,7 (positiv) | 2,2 (positiv) | 0,1 (negativ) | 0,5 (negativ) | 2.3 (positiv) |

Es zeigt sich, daß der monoklonale Antikörper MAK <p24>M-4B1/1 und der monoklonale Antikörper MAK <p24>M-6A9/5 jeweils bevorzugt für den Einsatz als R1, d.h. auf der Festphasenseite geeignet sind. Der monoklonale Antikörper MAK <p24>M-2E7/3 und der monoklonale Antikörper MAK <p24>M-6D9/4 sind bevorzugt für den Einsatz als R2, d.h. auf der Nachweisseite geeignet. Für eine effektive gleichzeitige Erkennung des p24 von HIV1 und HIV1-SubO werden alle vier Antikörper bevorzugt gemeinsam eingesetzt.

## Patentansprüche

1. Verfahren zur Diagnose einer HIV-Infektion mittels eines Immunoassays durch den spezifischen Nachweis von p24-Antigen von HIV1, HIV1-SubO und/oder p26-Antigen von HIV2, mindestens einem Antikörper gegen den env-Bereich von HIV1, HIV1-SubO und/oder HIV2, und mindestens einem Antikörper gegen den pol- und/oder gag-Bereich aus HIV1, HIV1-SubO und/oder HIV2, wobei der gag-Bereich nicht Sequenzen von p24/p26 umfasst, und wobei die Bestimmung der einzelnen HIV-Antigene und HIV-Antikörper als einzelner Test simultan erfolgt.

2. Verfahren nach Anspruch 1,
**dadurch gekennzeichnet,**
**dass** der Nachweis der HIV-Infektion mittels eines heterogenen Immunoassays erfolgt.

3. Verfahren nach Anspruch 1 oder 2,
**dadurch gekennzeichnet,**
**dass** für den Nachweis mindestens ein Rezeptor R1, der mit dem p24-Antigen von HIV1, HIV1-SubO und/oder dem p26-Antigen von HIV2 spezifisch bindefähig ist und an eine Festphase gebunden ist oder gebunden werden kann,
mindestens ein Rezeptor R2, der mit dem p24-Antigen von HIV1, HIV1-SubO und/oder dem p26-Antigen von HIV2 spezifisch bindefähig ist und
eine Markierung trägt, wobei die von R1 und R2 erkannten Epitope verschieden sind,
mindestens ein Rezeptor R3, der mit dem zu bestimmenden, gegen ein Antigen aus dem env-Bereich von HIV1-, HIV1-SubO- und/oder HIV2 gerichteten Antikörper spezifisch bindefähig ist und an eine Festphase gebunden ist oder gebunden werden kann,
mindestens ein Rezeptor R4, der mit dem zu bestimmenden, gegen ein Antigen aus dem env-Bereich von HIV1-, HIV1-SubO- und/oder HIV2 gerichteten Antikörper spezifisch bindefähig ist und eine Markierung trägt, mindestens ein Rezeptor R5, der mit dem zu bestimmenden, gegen ein Antigen aus dem pol- oder gag-Bereich von HIV1-, HIV1-SubO- und/oder HIV2 gerichteten Antikörper spezifisch bindefähig ist und an eine Festphase gebunden ist und gebunden werden kann,
mindestens ein Rezeptor R6, der mit dem zu bestimmenden, gegen ein Antigen aus dem pol- oder gag-Bereich von HIV1-, HIV1-SubO- und/oder HIV2 gerichteten Antikörper spezifisch bindefähig ist und eine Markierung trägt, eingesetzt werden, gegebenenfalls Trennung der festen von der flüssigen Phase und Bestimmung der Markierung in einer der beiden Phasen.

4. Verfahren nach Anspruch 2 oder 3,
**dadurch gekennzeichnet,**
**dass** die Rezeptoren R1 und R2 mit dem p24-Antigen von HIV1 spezifisch bindefähig sind, die Rezeptoren R3 und R4 mit dem zu bestimmenden, gegen ein Antigen aus dem env-Bereich gerichteten HIV1-Antikörper spezifisch bindefähig sind und die Rezeptoren R5 und R6 mit dem zu bestimmenden, gegen ein Antigen aus dem gag- oder pol-Bereich gerichteten HIV1-Antikörper spezifisch bindefähig sind.

5. Verfahren nach Anspruch 3 oder 4,
**dadurch gekennzeichnet,**
**dass** die Rezeptoren R5 und R6 mit dem zu bestimmenden, gegen ein Antigen aus dem pol-Bereich gerichteten HIV1-Antikörper spezifisch bindefähig sind.

6. Verfahren nach einem der Ansprüche 3 bis 5,
**dadurch gekennzeichnet,**
**dass** die Rezeptoren R5 und R6 mit gegen die HIV-Reverse Transkriptase gerichteten Antikörpern spezifisch bindefähig sind.

7. Verfahren nach einem der Ansprüche 3 bis 5,
**dadurch gekennzeichnet,**
**dass** die Rezeptoren R5 und R6 mit gegen das gag-p17-Antigen
gerichteten Antikörpern spezifisch bindefähig sind.

8. Verfahren nach einem der Ansprüche 1 bis 7,
**dadurch gekennzeichnet,**
**dass** auch eine Infektion mit HIV1-Subtyp O detektiert wird.

9. Verfahren nach einem der Ansprüche 1 bis 8,
**dadurch gekennzeichnet,**
**dass** auch eine Infektion mit allen HIV1-Subtypen der Gruppe M detektiert wird.

10. Verfahren nach einem der Ansprüche 1 bis 9,
**dadurch gekennzeichnet,**
**dass** die Rezeptoren R1 und R2 jeweils Gemische aus verschiedenen Rezeptoren sind, die unterschiedliche Epitope des p24-Antigens von HIV1, HIV1-SubO und/oder unterschiedliche Epitope des p26-Antigens von HIV2 erkennen.

11. Verwendung eines monoklonalen Antikörpers gegen HIV1 p24-Antigen, der die p24-Epitope gemäß SEQ ID NO: 3, 9, 17, 31 oder 34 spezifisch bindet, in einem Verfahren nach einem der Ansprüche 1 bis 10.

12. Monoklonale Antikörper gegen HIV1 p24-Antigen, die von den Zelllinien MAK<p24>M-6A9/5 (Hinterlegungsnr. DSM ACC2310), MAK<p24>M-4B1/1 (Hinterlegungsnr. DSM ACC2299), MAK<p24>M-6D9/4 (Hinterlegungsnr. DSM ACC2300) oder MAK<p24>M-2E7/3 (Hinterlegungsnr. DSM ACC2301) produziert werden.

13. Verwendung monoklonaler Antikörper gegen HIV1 p24-Antigen, die in äquivalenter Weise an p24 binden wie die monoklonalen Antikörper, die von den Zelllinien MAK<p24>M-6A9/5 (Hinterlegungsnr. DSM ACC2310), MAK<p24>M-4B1/1 (Hinterlegungsnr. DSM ACC2299), MAK<p24>M-6D9/4 (Hinterlegungsnr. DSM ACC2300) oder MAK<p24>M-2E7/3 (Hinterlegungsnr. DSM ACC2301) produziert werden, in einem Verfahren nach einem der Ansprüche 1 bis 10.

14. Verwendung monoklonaler Antikörper gegen HIV1 p24-Antigen, die an die gleichen Epitope binden wie die monoklonalen Antikörper, die von den Zelllinien MAK<p24>M-6A9/5 (Hinterlegungsnr. DSM ACC2310), MAK<p24>M-4B1/1 (Hinterlegungsnr. DSM ACC2299), MAK<p24>M-6D9/4 (Hinterlegungsnr. DSM ACC2300) oder MAK<p24>M-2E7/3 (Hinterlegungsnr. DSM ACC2301) produziert werden, in einem Verfahren nach einem der Ansprüche 1 bis 10.

15. Verwendung mindestens eines monoklonalen Antikörpers gemäß Anspruch 12 in einem diagnostischen Test zum Nachweis einer HIV-Infektion.

16. Verwendung mindestens eines monoklonalen Antikörpers gemäß Anspruch 12 in einem diagnostischen Test in einem Verfahren gemäß Anspruch 1 bis 10 zum Nachweis einer HIV-Infektion.

17. Reagenzienkit zum Nachweis einer HIV-Infektion, enthaltend mindestens einen Rezeptor R1, der mit dem p24-Antigen von HIV1, HIV1-SubO und/oder dem p26-Antigen von HIV2 spezifisch bindefähig ist und an eine Festphase gebunden ist oder gebunden werden kann,
mindestens einen Rezeptor R2, der mit dem p24-Antigen von HIV1, HIV1-SubO und/oder dem p26-Antigen von HIV2 spezifisch bindefähig ist und eine Markierung trägt, wobei die von R1 und R2 erkannten Epitope verschieden sind,
mindestens einen Rezeptor R3, der mit dem zu bestimmenden, gegen ein Antigen aus dem env-Bereich von HIV1-, HIV1-SubO- und/oder HIV2 gerichteten Antikörper spezifisch bindefähig ist und an eine Festphase gebunden ist oder gebunden werden kann,
mindestens einen Rezeptor R4, der mit dem zu bestimmenden, gegen ein Antigen aus dem env-Bereich von HIV1-, HIV1-SubO- und/oder HIV2 gerichteten Antikörper spezifisch bindefähig ist und eine Markierung trägt, mindestens einen Rezeptor R5, der mit dem zu bestimmenden, gegen ein Antigen aus dem pol- oder gag-Bereich von HIV1-, HIV1-SubO- und/oder HIV2 gerichteten Antikörper spezifisch bindefähig ist und an eine Festphase gebunden ist oder gebunden werden kann,
mindestens einen Rezeptor R6, der mit dem zu bestimmenden, gegen ein Antigen aus dem pol- oder gag-Bereich von HIV1-, HIV1-SubO- und/oder HIV2 gerichteten Antikörper spezifisch bindefähig ist und eine Markierung trägt sowie gegebenenfalls weitere übliche Hilfsstoffe, wobei der gag-Bereich nicht Sequenzen von p24/p26 umfasst.

18. Reagenzienkit gemäß Anspruch 17,
**dadurch gekennzeichnet,**
**dass** als Rezeptoren R1 und R2 die Antikörper, die von den Zelllinien MAK<p24>M-6A9/5 (Hinterlegungsnr. DSM ACC2310), MAK<p24>M-4B1/1 (Hinterlegungsnr. DSM ACC2299), MAK<p24>M-6D9/4 (Hinterlegungsnr. DSM ACC2300) oder MAK<p24>M-2E7/3 (Hinterlegungsnr. DSM ACC2301) produziert werden, eingesetzt werden.

19. Teststreifen, enthaltend mindestens einen Rezeptor R1, der mit dem p24-Antigen von HIV1, HIV1-SubO und/oder dem p26-Antigen von HIV2 spezifisch bindefähig ist und an eine Festphase gebunden ist oder gebunden werden kann,
mindestens einen Rezeptor R2, der mit dem p24-Antigen von HIV1, HIV1-SubO und/oder dem p26-Antigen von HIV2 spezifisch bindefähig ist und eine Markierung trägt, wobei die von R1 und R2 erkannten Epitope verschieden sind,
mindestens einen Rezeptor R3, der mit dem zu bestimmenden, gegen ein Antigen aus dem env-Bereich von HIV1-, HIV1-SubO- und/oder HIV2 gerichteten Antikörper spezifisch bindefähig ist und an eine Festphase gebunden ist oder gebunden werden kann,
mindestens einen Rezeptor R4, der mit dem zu bestimmenden, gegen ein Antigen aus dem env-Bereich von HIV1-, HIV1-SubO- und/oder HIV2 gerichteten Antikörper spezifisch bindefähig ist und eine Markierung trägt, mindestens einen Rezeptor R5, der mit dem zu bestimmenden, gegen ein Antigen aus dem pol- oder gag-Bereich von HIV1-, HIV1-SubO- und/oder HIV2 gerichteten Antikörper spezifisch bindefähig ist und an eine Festphase gebunden ist oder gebunden werden kann,
mindestens einen Rezeptor R6, der mit dem zu bestimmenden, gegen ein Antigen aus dem pol- oder gag-Bereich von HIV1- und/oder HIV2 gerichteten Antikörper spezifisch bindefähig ist und eine Markierung trägt sowie gegebenenfalls weitere übliche Hilfsstoffe, wobei der gag-Bereich nicht Sequenzen von p24/p26 umfasst.

## Claims

1. Method for diagnosing an HIV infection by means of an immunoassay through the specific detection of the p24 antigen of HIV1, HIV1-SubO and/or the p26 antigen of HIV2, at least one antibody against the env region of HIV1, HIV1-SubO and/or HIV2, and at least one antibody against the pol and/or gag region of HIV1, HIV1-Sub-O and/or HIV2, wherein the gag region does not include sequences of p24/p26 and wherein the determination of the individual HIV antigens and HIV antibodies takes place simultaneously as a single test.

2. Method according to claim 1,
**characterised in that**
the detection of the HIV infection takes place by means of a heterogeneous immunoassay.

3. Method according to claim 1 or 2,
**characterised in that**
for the detection at least one receptor R1 that is capable of specifically binding to the p24 antigen of HIV1, HIV1-SubO and/or to the p26 antigen of HIV2 and is bound to or is capable of binding to a solid phase, at least one receptor R2 that is capable of specifically binding to the p24 antigen of HIV1, HIV1-SubO and/or to the p26 antigen of HIV2 and carries a label, wherein the epitopes recognised by R1 and R2 are different,
at least one receptor R3 that is capable of specifically binding to the antibody to be determined that is directed against an antigen from the env region of HIV1, HIV1-SubO and/or HIV2 and is bound to or is capable of binding to a solid phase,
at least one receptor R4 that is capable of specifically binding to the antibody to be determined that is directed against an antigen from the env region of HIV1, HIV1-SubO and/or HIV2 and carries a label, at least one receptor R5 that is capable of specifically binding to the antibody to be determined that is directed against an antigen from the pol or gag region of HIV1, HIV1-SubO and/or HIV2 and is bound to and is capable of binding to a solid phase,
at least one receptor R6 that is capable of specifically binding to the antibody to be determined that is directed against an antigen from the pol or gag region of HIV1, HIV1-SubO and/or HIV2 and carries a label are used, with optional separation of the solid phase from the liquid phase and determination of the label in one of the two phases.

4. Method according to claim 2 or 3,
**characterised in that**
the receptors R1 and R2 are capable of specifically binding to the p24 antigen of HIV1, the receptors R3 and R4 are capable of specifically binding to the HIV1 antibody to be determined that is directed against an antigen from the env region and the receptors R5 and R6 are capable of specifically binding to the HIV1 antibody to be determined that is directed against an antigen from the gag or pol region.

5. Method according to claim 3 or 4,
**characterised in that**
the receptors R5 and R6 are capable of specifically binding to the HIV1 antibody to be determined that is directed against an antigen from the pol region.

6. Method according to one of claims 3 to 5,
**characterised in that**
the receptors R5 and R6 are capable of specifically binding to antibodies that are directed against the HIV reverse transcriptase.

7. Method according to one of claims 3 to 5,
**characterised in that**
the receptors R5 and R6 are capable of specifically binding to antibodies that are directed against the gag-p17 antigen.

8. Method according to one of claims 1 to 7,
**characterised in that**
an infection with HIV1 subtype O is also detected.

9. Method according to one of claims 1 to 8,
**characterised in that**
an infection with all HIV1 subtypes of group M is also detected.

10. Method according to one of claims 1 to 9,
**characterised in that**
the receptors R1 and R2 are each mixtures of various receptors that recognise different epitopes of the p24 antigen of HIV1, HIV1-SubO and/or different epitopes of the p26 antigen of HIV2.

11. Use of a monoclonal antibody against HIV1 p24 antigen that specifically binds the p24 epitopes according to SEQ ID NO: 3, 9, 17, 31 or 34, in a method according to one of claims 1 to 10.

12. Monoclonal antibodies against HIV1 p24 antigen that are produced by the cell lines MAK<p24>M-6A9/5 (deposit number DSM ACC2310), MAK<p24>M-4B1/1 (deposit number DSM ACC2299), MAK<p24>M-6D9/4 (deposit number DSM ACC2300) or MAK<p24>M-2E7/3 (deposit number DSM ACC2301).

13. Use of monoclonal antibodies against HIV1 p24 antigen that bind to p24 in an equivalent way to the monoclonal antibodies produced by the cell lines MAK<p24>M-6A9/5 (deposit number DSM ACC2310), MAK<p24>M-4B1/1 (deposit number DSM ACC2299), MAK<p24>M-6D9/4 (deposit number DSM ACC2300) or MAK<p24>M-2E7/3 (deposit number DSM ACC2301), in a method according to one of claims 1 to 10.

14. Use of monoclonal antibodies against HIV1 p24 antigen that bind to the same epitopes as the monoclonal antibodies that are produced by the cell lines
MAK<p24>M-6A9/5 (deposit number DSM ACC2310),
MAK<p24>M-4B1/1 (deposit number DSM ACC2299),
MAK<p24>M-6D9/4 (deposit number DSM ACC2300) or
MAK<p24>M-2E7/3 (deposit number DSM ACC2301), in a method according to one of claims 1 to 10.

15. Use of at least one monoclonal antibody according to claim 12 in a diagnostic test for the detection of an HIV infection.

16. Use of at least one monoclonal antibody according to claim 12 in a diagnostic test in a method according to claim 1 to 10 for the detection of an HIV infection.

17. Reagent kit for the detection of an HIV infection, containing at least one receptor R1 that is capable of specifically binding to the p24 antigen of HIV1, HIV1-SubO and/or to the p26 antigen of HIV2 and is bound to or is capable of binding to a solid phase,
at least one receptor R2 that is capable of specifically binding to the p24 antigen of HIV1, HIV1-SubO and/or to the p26 antigen of HIV2 and carries a label, wherein the epitopes recognised by R1 and R2 are different,
at least one receptor R3 that is capable of specifically binding to the antibody to be determined that is directed against an antigen from the env region of HIV1, HIV1-SubO and/or HIV2 and is bound to or is capable of binding to a solid phase,
at least one receptor R4 that is capable of specifically binding to the antibody to be determined that is directed against an antigen from the env region of HIV1, HIV1-SubO and/or HIV2 and carries a label, at least one receptor R5 that is capable of specifically binding to the antibody to be determined that is directed against an antigen from the pol or gag region of HIV1, HIV1-SubO and/or HIV2 and is bound to or is capable of binding to a solid phase, at least one receptor R6 that is capable of specifically binding to the antibody to be determined that is directed against an antigen from the pol or gag region of HIV1, HIV1-SubO and/or HIV2 and carries a label, as well as optionally further conventional auxiliary substances, wherein the gag region does not include sequences of p24/p26.

18. Reagent kit according to claim 17,
**characterised in that**
the antibodies produced by the cell lines MAK<p24>M-6A9/5 (deposit number DSM ACC2310), MAK<p24>M-4B1/1 (deposit number DSM ACC2299), MAK<p24>M-6D9/4 (deposit number DSM ACC2300) or MAK<p24>M-2E7/3 (deposit number DSM ACC2301) are used as the receptors R1 and R2.

19. Test strips containing at least one receptor R1 that is capable of specifically binding to the p24 antigen of HIV1, HIV1-SubO and/or to the p26 antigen of HIV2 and is bound to or is capable of binding to a solid phase, at least one receptor R2 that is capable of specifically binding to the p24 antigen of HIV1, HIV1-SubO and/or to the p26 antigen of HIV2 and carries a label, wherein the epitopes recognised by R1 and R2 are different,
at least one receptor R3 that is capable of specifically binding to the antibody to be determined that is directed against an antigen from the env region of HIV1, HIV1-SubO and/or HIV2 and is bound to or is capable of binding to a solid phase,
at least one receptor R4 that is capable of specifically binding to the antibody to be determined that is directed against an antigen from the env region of HIV1, HIV1-SubO and/or HIV2 and carries a label,
at least one receptor R5 that is capable of specifically binding to the antibody to be determined that is directed against an antigen from the pol or gag region of HIV1, HIV1-SubO and/or HIV2 and is bound to or is capable of binding to a solid phase,
at least one receptor R6 that is capable of specifically binding to the antibody to be determined that is directed against an antigen from the pol or gag region of HIV1 and/or HIV2 and carries a label, as well as optionally further conventional auxiliary substances, wherein the gag region does not include sequences of p24/p26.

## Revendications

1. Procédé pour le diagnostic d'une infection à VIH à l'aide d'un immunodosage via le décèlement spécifique de l'antigène p24 de VIH1, du sous-type O de VIH1 et/ou de l'antigène p26 de VIH2, d'au moins un anticorps dirigé contre le domaine env de VIH 1, du sous-type O de VIH1 et/ou de VIH2 et d'au moins un anticorps dirigé contre le domaine pol et/ou gag de VIH 1, du sous-type O de VIH1 et/ou de VIH2, dans lequel le domaine gag ne comprend pas de séquences de p24/p26, et dans lequel la détermination des antigènes VIH et des anticorps anti-VIH individuels a lieu de manière simultanée sous la forme d'un test individuel.

2. Procédé selon la revendication 1, **caractérisé en ce que** le décèlement de l'infection à VIH a lieu à l'aide d'un immunodosage hétérogène.

3. Procédé selon la revendication 1 ou 2, **caractérisé en ce qu'**on met en oeuvre, pour le décèlement au moins un récepteur R1 qui manifeste une capacité de liaison spécifique avec l'antigène p24 de VIH1, du sous-type O de VIH1 et/ou avec l'antigène p26 de VIH2 et qui est lié ou qui peut être lié à une phase solide,
au moins un récepteur R2 qui manifeste une capacité de liaison spécifique avec l'antigène p24 de VIH1, du sous-type O de VIH1 et/ou avec l'antigène p26 de VIH2 et qui porte un marqueur, les épitopes reconnus par R1 et R2 étant différents,
au moins un récepteur R3 qui manifeste une capacité de liaison spécifique avec l'anticorps à déterminer, dirigé contre un antigène issu du domaine env de VIH1, du sous-type O de VIH1 et/ou de VIH2 et qui est lié ou qui peut être lié à une phase solide,
au moins un récepteur R4 qui manifeste une capacité de liaison spécifique avec l'anticorps à déterminer, dirigé contre un antigène issu du domaine env de VIH1, du sous-type O de VIH1 et/ou de VIH2 et qui porte un marqueur,
au moins un récepteur R5 qui manifeste une capacité de liaison spécifique avec l'anticorps à déterminer, dirigé contre un antigène issu du domaine pol ou gag de VIH1, du sous-type O de VIH1 et/ou de VIH2 et qui est lié ou qui peut être lié à une phase solide,
au moins un récepteur R6 qui manifeste une capacité de liaison spécifique avec l'anticorps à déterminer, dirigé contre un antigène issu du domaine pol ou gag de VIH1, du sous-type O de VIH1 et/ou de VIH2 et qui porte un marqueur, en procédant, le cas échéant, à une séparation de la phase solide par rapport à la phase liquide et à une détermination du marqueur dans une des deux phases.

4. Procédé selon la revendication 2 ou 3, **caractérisé en ce que** les récepteurs R1 et R2 manifestent une capacité de liaison spécifique avec l'antigène p24 de VIH1, les récepteurs R3 et R4 manifestent une capacité de liaison spécifique avec l'anticorps à déterminer, dirigé contre un antigène issu du domaine env de VIH1 et les récepteurs R5 et R6 manifestent une capacité de liaison spécifique avec l'anticorps anti-VIH1 à déterminer, dirigé contre un antigène issu du domaine gag ou pol.

5. Procédé selon la revendication 3 ou 4, **caractérisé en ce que** les récepteurs R5 et R6 manifestent une capacité de liaison spécifique avec l'anticorps anti-VIH1 à déterminer, dirigé contre un antigène issu du domaine pol.

6. Procédé selon l'une quelconque des revendications 3 à 5, **caractérisé en ce que** les récepteurs R5 et R6 manifestent une capacité de liaison spécifique avec des anticorps dirigés contre la transcriptase inverse de VIH.

7. Procédé selon l'une quelconque des revendications 3 à 5, **caractérisé en ce que** les récepteurs R5 et R6 manifestent une capacité de liaison spécifique avec des anticorps dirigés contre l'antigène gag-p17.

8. Procédé selon l'une quelconque des revendications 1 à 7, **caractérisé en ce qu'**on détecte également une infection avec le sous-type O de VIH1.

9. Procédé selon l'une quelconque des revendications 1 à 8, **caractérisé en ce qu'**on détecte également une infection avec tous les sous-types du groupe M de VIH1.

10. Procédé selon l'une quelconque des revendications 1 à 9, **caractérisé en ce que** les récepteurs R1 et R2 représentent à chaque fois des mélanges de différents récepteurs qui reconnaissent des épitopes différents de l'antigène p24 de VIH1, du sous-type O de VIH1 et/ou des épitopes différents de l'antigène p26 de VIH2.

11. Utilisation d'un anticorps monoclonal dirigé contre l'antigène p24 de VIH-1, qui se lie de manière spécifique aux épitopes de p24 conformément aux SEQ ID NO: 3, 9, 17, 31 ou 34, dans un procédé selon l'une quelconque des revendications 1 à 10.

12. Anticorps monoclonaux dirigés contre l'antigène p24 de VIH1, qui sont produits par les lignées cellulaires MAK<p24>M-6A9/5 (numéro de dépôt DSM ACC2310), MAK<p24>M-4B1/1 (numéro de dépôt DSM ACC2299), MAK<p24>M-6D9/4 (numéro de dépôt DSM ACC2300) ou MAK<p24>M2E7/3 (numéro de dépôt DSM ACC2301).

13. Utilisation d'anticorps monoclonaux dirigés contre l'antigène p24 de VIH1, dont la liaison à p24 est équivalente à celle des anticorps monoclonaux qui sont produits par les lignées cellulaires MAK<p24>M-6A9/5 (numéro de dépôt DSM ACC2310), MAK<p24>M-4B1/1 (numéro de dépôt DSM ACC2299), MAK<p24>M-6D9/4 (numéro de dépôt DSM ACC2300) ou MAK<p24>M2E7/3 (numéro de dépôt DSM ACC2301), dans un procédé selon l'une quelconque des revendications 1 à 10.

14. Utilisation d'anticorps monoclonaux dirigés contre l'antigène p24 de VIH1, dont les épitopes auxquels ils se lient sont identiques à ceux auxquels se lient les anticorps monoclonaux qui sont produits par les lignées cellulaires MAK<p24>M-6A9/5 (numéro de dépôt DSM ACC2310), MAK<p24>M-4B1/1 (numéro de dépôt DSM ACC2299), MAK<p24>M-6D9/4 (numéro de dépôt DSM ACC2300) ou MAK<p24>M2E7/3 (numéro de dépôt DSM ACC2301) dans un procédé selon l'une quelconque des revendications 1 à 10.

15. Utilisation d'au moins un anticorps monoclonal selon la revendication 12, dans un essai diagnostique pour le décèlement d'une infection à VIH.

16. Utilisation d'au moins un anticorps monoclonal selon la revendication 12, dans un essai diagnostique dans un procédé selon les revendications 1 à 10 pour le décèlement d'une infection à VIH.

17. Nécessaire de réactifs pour le décèlement d'une infection à VIH, contenant au moins un récepteur R1 qui manifeste une capacité de liaison spécifique avec l'antigène p24 de VIH1, du sous-type O de VIH1 et/ou avec l'antigène p26 de VIH2 et qui est lié ou qui peut être lié à une phase solide,
au moins un récepteur R2 qui manifeste une capacité de liaison spécifique avec l'antigène p24 de VIH1, du sous-type O de VIH1 et/ou avec l'antigène p26 de VIH2 et qui porte un marqueur, les épitopes reconnus par R1 et R2 étant différents,
au moins un récepteur R3 qui manifeste une capacité de liaison spécifique avec l'anticorps à déterminer, dirigé contre un antigène issu du domaine env de VIH1, du sous-type O de VIH1 et/ou de VIH2 et qui est lié ou qui peut être lié à une phase solide,
au moins un récepteur R4 qui manifeste une capacité de liaison spécifique avec l'anticorps à déterminer, dirigé contre un antigène issu du domaine env de VIH1, du sous-type O de VIH1 et/ou de VIH2 et qui porte un marqueur,
au moins un récepteur R5 qui manifeste une capacité de liaison spécifique avec l'anticorps à déterminer, dirigé contre un antigène issu du domaine pol ou gag de VIH1, du sous-type O de VIH1 et/ou de VIH2 et qui est lié ou qui peut être lié à une phase solide,
au moins un récepteur R6 qui manifeste une capacité de liaison spécifique avec l'anticorps à déterminer, dirigé contre un antigène issu du domaine pol ou gag VIH1, du sous-type O de VIH1 et/ou de VIH2 et qui porte un marqueur, et le cas échéant, d'autres adjuvants habituels, dans lequel le domaine gag ne comprend pas de séquences de p24/p26.

18. Nécessaire de réactifs selon la revendication 17, **caractérisé en ce qu'**on met en oeuvre, à titre de récepteurs R1 et R2, les anticorps qui sont produits par les lignées cellulaires MAK<p24>M-6A9/5 (numéro de dépôt DSM ACC2310), MAK<p24>M-4B1/1 (numéro de dépôt DSM ACC2299), MAK<p24>M-6D9/4 (numéro de dépôt DSM ACC2300) ou MAK<p24>M2E7/3 (numéro de dépôt DSM ACC2301).

19. Bandelette réactive contenant au moins un récepteur R1 qui manifeste une capacité de liaison spécifique avec l'antigène p24 de VIH1, du sous-type O de VIH1 et/ou avec l'antigène p26 de VIH2 et qui est lié ou qui peut être lié à une phase solide,
au moins un récepteur R2 qui manifeste une capacité de liaison spécifique avec l'antigène p24 de VIH1, du sous-type O de VIH1 et/ou avec l'antigène p26 de VIH2 et qui porte un marqueur, les épitopes reconnus par R1 et R2 étant différents,
au moins un récepteur R3 qui manifeste une capacité de liaison spécifique avec l'anticorps à déterminer, dirigé contre un antigène issu du domaine env de VIH1, du sous-type O de VIH1 et/ou de VIH2 et qui est lié ou qui peut être lié à une phase solide,
au moins un récepteur R4 qui manifeste une capacité de liaison spécifique avec l'anticorps à déterminer, dirigé contre un antigène issu du domaine env de VIH1, du sous-type O de VIH1 et/ou de VIH2 et qui porte un marqueur,
au moins un récepteur R5 qui manifeste une capacité de liaison spécifique avec l'anticorps à déterminer, dirigé contre un antigène issu du domaine pol ou gag de VIH1, du sous-type O de VIH1 et/ou de VIH2 et qui est lié ou qui peut être lié à une phase solide,
au moins un récepteur R6 qui manifeste une capacité de liaison spécifique avec l'anticorps à déterminer, dirigé contre un antigène issu du domaine pol ou gag de VIH1 et/ou de VIH2 et qui porte un marqueur, et le cas échéant, d'autres adjuvants habituels, dans lequel le domaine gag ne comprend pas de séquences de p24/p26.
